(19) Europäisches Patentamt
European Patent Office
Office européen des brevets

(11) EP 1 805 202 B1

(12) **EUROPEAN PATENT SPECIFICATION**

(45) Date of publication and mention of the grant of the patent:
**30.12.2009 Bulletin 2009/53**

(21) Application number: **05824103.5**

(22) Date of filing: **27.10.2005**

(51) Int Cl.:
***C07J 43/00*** *(2006.01)* ***C07D 413/12*** *(2006.01)*
***C07D 267/00*** *(2006.01)* ***A61K 31/58*** *(2006.01)*
***A61P 29/00*** *(2006.01)*

(86) International application number:
**PCT/IB2005/003213**

(87) International publication number:
**WO 2006/046123 (04.05.2006 Gazette 2006/18)**

(54) **CONJUGATES WITH ANTI-INFLAMMATORY ACTIVITY**

KONJUGATE MIT ENTZÜNDUNGSHEMMENDER AKTIVITÄT

CONJUGUES A ACTIVITE ANTI-INFLAMMATOIRE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **27.10.2004 US 623154 P**

(43) Date of publication of application:
**11.07.2007 Bulletin 2007/28**

(60) Divisional application:
**09075144.7**

(73) Proprietor: **GlaxoSmithKline istrazivacki centar Zagreb d.o.o.**
**10000 Zagreb (HR)**

(72) Inventors:
- **MERCEP, Mladen**
**GlaxoSmithKline istrazivacki centra Zagreb d.o.o.**
**10000 Zagreb (HR)**
- **MESIC, Milan**
**GlaxoSmithKline istrazivacki centra Zagreb d.o.o.**
**10000 Zagreb (HR)**
- **TOMASKOVIC, Linda**
**GlaxoSmithKline istrazivacki centra Zagreb d.o.o.**
**10000 Zagreb (HR)**
- **MARKOVIC, Stribor**
**GlaxoSmithKline istrazivacki centra Zagreb d.o.o.**
**10000 Zagreb (HR)**
- **POLJAK, Visnja**
**GlaxoSmithKline istrazivacki centra Zagreb d.o.o.**
**10000 Zagreb (HR)**
- **SIJAN, Gordana**
**GlaxoSmithKline istrazivacki centra Zagreb d.o.o.**
**10000 Zagreb (HR)**
- **SELMANI, Selvira**
**GlaxoSmithKline istrazivacki centra Zagreb d.o.o.**
**10000 Zagreb (HR)**

(74) Representative: **Crawley, Karen Anne et al**
**GlaxoSmithKline**
**Corporate Intellectual Property (CN9.25.1)**
**980 Great West Road**
**Brentford, Middlesex TW8 9GS (GB)**

(56) References cited:
**WO-A-02/055531** **WO-A-03/070174**
**WO-A-2004/094449**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 805 202 B1

## Description

### Background of the Invention

Anti-inflammatory medicaments can be classified into those of steroid and of nonsteroidal type. Steroid anti-inflammatory compounds are still the most effective ones in the treatment of inflammatory diseases and conditions such as: asthma, inflammatory nasal diseases such as allergic rhinitis, nasal polyps, intestinal diseases such as Crohn's disease, colitis, ulcerative colitis, dermatological inflammations such as eczema, psoriasis, allergic dermatitis, neurodermatitis, pruritis, conjunctivitis and rheumatoid arthritis. In addition to excellent potency and effectiveness, medicaments of this type also possess numerous unfavourable side-effects, (e.g. disturbance of carbohydrate metabolism, decreased calcium resorption, decreased excretion of endogenous corticosteroids and disturbance of physiological functions of the pituitary gland, adrenal cortex and thymus. Steroids present on the market are highly effective against inflammatory conditions and processes whereas their systemic side-effects are diminished. Patent applications WO 94/13690; 94/14834; 92/13872 and 92/13873 describe the so-called "soft" steroids or hydrolysable corticosteroids designed for topical application at the inflammation site, whereas their systemic side-effects are diminished due to the hydrolysis in the serum, wherein the active steroid very rapidly hydrolyses into the inactive form. An ideal steroid, however, without unfavourable effects in a long-term and continuous treatment as required for the control of diseases such as asthma or Crohn's disease has yet to be found, so that there are intense efforts on the discovery and development of steroids with improved therapeutic profile.

Macrolide antibiotics accumulate preferentially within different cells of subjects, especially within phagocyte cells such as mononuclear peripheral blood cells, and peritoneal and alveolar macrophages. (Gladue, R. P. et al, Antimicrob. Agents Chemother. 1989, 33, 277-282; Olsen, K. M. et al, Antimicrob. Agents Chemother. 1996, 40, 2582-2585*)*. Inflammatory effects of some macrolides have been described in the literature, although their effects are relatively weak. For example, the anti-inflammatory effect of erythromycin derivatives (J. Antimicrob. Chemother. 1998, 41, 37-46; WO 00/42055) and azithromycin derivatives has been described (EP 0283055). Anti-inflammatory effects of some macrolides are also known from *in vitro* and *in vivo* studies in experimental animal models such as in zymosan-induced peritonitis in mice *(*J. Antimicrob. Chemother. 1992, 30, 339-348) and endotoxin-induced neutrophil accumulation in rat trachea *(*J. Immunol. 1997, 159, 3395-4005). The modulating effect of macrolides upon cytokines such as interleukin 8 (IL-8) *(*Am. J. Respir. Crit. Care. Med. 1997, 156, 266-271) and interleukin 5 (IL-5) (EP 0775489 and EP771564) is known as well.

International Publication No. WO 02/055531 A1 discloses conjugate compounds represented by the Formula **II:**

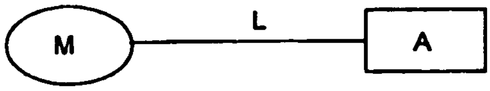


**II**

wherein **M** represents a macrolide subunit possessing the property of accumulation in inflammatory cells, **A** represents an anti-inflammatory subunit that can be steroid or nonsteroid, and **L** represents a linker molecule linking **M** and **A,** (b) their pharmacologically acceptable salts, prodrugs and solvates, (c) processes and intermediates for their preparation, and (d) their use in the treatment of inflammatory diseases and conditions in humans and animals. In WO 02/05531, the conjugate steroid-macrolide compounds are mostly linked with the steroid subunit at the N/9a-position of macrolide ring.

U.S. Published Application 2004 0014685 and International Publication No. WO 04/005310 A2 relate to compounds represented by Formula **III.**

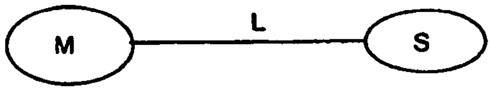


**III**

wherein M represents a macrolide subunit (macrolide moiety) derived from macrolide possessing the property of accumulation in inflammatory cells, S represents a steroid subunit derived from a steroid drug with anti-inflammatory activity and L represents a linker molecule linking M and S to their pharmaceutically acceptable salts and solvates processes and intermediates for their preparation and to their use in the treatment of inflammatory diseases and conditions in

humans and animals.

US Published Application 20040077612 relates to new compounds represented by Formula **IV.**

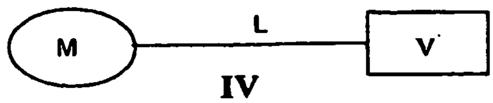


**IV**

wherein M represents a macrolide subunit (macrolide moiety) derived from macrolide possessing the property of accumulation in inflammatory cells, V represents an anti-inflammatory steroid or non steroid subunit or an anti neoplastic or antiviral subunit and L represents a linking group covalently linking M and V to their pharmaceutically acceptable salts and solvates processes and intermediates for their preparation and to their use in the treatment of inflammatory diseases and conditions in humans and animals.

US Published Application 2004 0097434 and International Publication No. WO 04/005309 relates to new compounds represented by formula **V**.

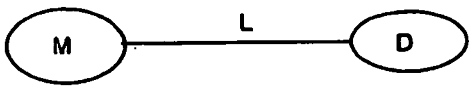


**V**

wherein M represents a macrolide subunit (macrolide moiety) derived from macrolide possessing the property of accumulation in inflammatory cells, D represents a nonsteroidal subunit (nonsteroidal moiety) derived from a nonsteroid drug with anti-inflammatory, analgesic and/or antipyretic activity (NSAID) and L represents a linking group covalent linking M and D to their pharmaceutically acceptable salts and solvates processes and intermediates for their preparation and to their use in the treatment of inflammatory diseases and conditions in humans and animals.

US Published Application 20050080003 describes yet further conjugate compounds having a steroid or non-steroidal anti-inflammatory subunit **D** linked via the chain L to position N/9a of an aglycone type macrolide subunit.

US Published Application 20040087517 and International Publication WO2003/070174 disclose a conjugate of (i) a "transportophore" and (ii) a "non-antibiotic therapeutic agent" covalently linked by a bond or a linker incorporating the transportophore. The transportophore and conjugate must have an immune selectivity ratio of at least 2. "Transportophore" is broadly defined as a compound, a portion of which resembles and is recognized as a substrate for transport protein(s).

However, there is still need for novel anti-inflammatory conjugates of macrolides and steroids having therapeutic action.

## Summary of the Invention

The present invention relates to the compound represented by the Formula **Ia**:

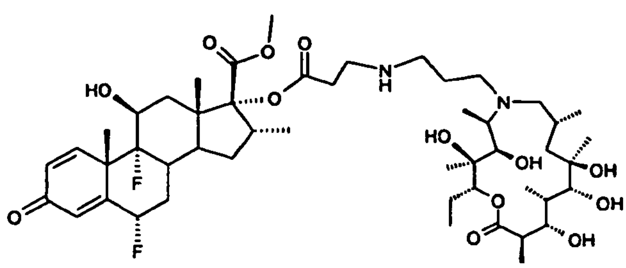

**Ia**

The present disclosure relates to: a) new compounds represented by the structure **I:**

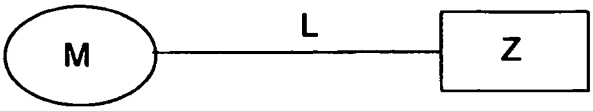


**I**

wherein **M** represents a macrolide subunit derived from macrolides, possessing the property of accumulation in inflammatory cells, **Z** represents either a steroid subunit or nonsteroidal subunit derived from nonsteroidal anti-inflammatory drugs (NSAID), and **L** represents a chain linking **M** and **Z;** b) their pharmacologically acceptable salts and solvates; c) processes and intermediates for their preparation and d) their activity and use in the treatment of inflammatory diseases and conditions in humans and animals. Specifically the macrolide subunit is an 9-deoxo-9-dihydro-9a-aza-9a-homoerythronolide **A** or an azithromycin aglycone subunit and the linkage to **Z** is effected via the linker **L** through the hydroxy group at position C/11 or through the nitrogen at position 9a of the aglycone ring. Also, specifically the macrolide subunit is an 9-deoxo-9-dihydro-9a-aza-9a-homoerythronolide **A** or an azithromycin aglycone subunit and **Z** is steroid subunit and the linkage to M is effected via the linker L through the 17α-hydroxy group.

**Detailed Description of the Invention**

A characteristic of compounds represented by Formula I is selective accumulation in target organs and cells in the above mentioned inflammatory diseases and conditions. These pharmacokinetic properties enable the compounds represented by Formula I to act at the inflammation site in inflammation cells by inhibiting the production of inflammation mediators. In such a manner, the unfavourable systemic side-effects of corticosteroids or non-steroidal anti-inflammatory molecules are avoided and the therapeutic action of either the steroid or the NSAID moiety is targeted to the area where it is most needed. Following local or systemic application molecules rapidly accumulate in inflammation cells wherein they act by inhibiting the production of cytokines and chemokines and/or other inflammatory mediators thus suppressing the inflammation.

According to the known and established state of the art, the compound represented by Formula **Ia,**

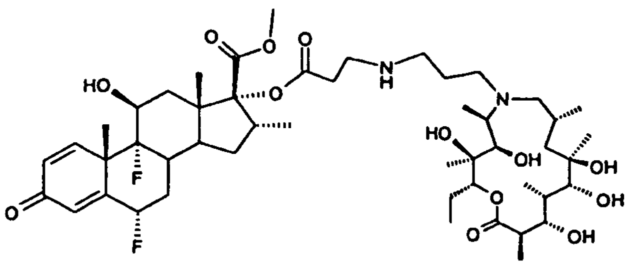

**Ia**

which is the object of the present invention, its pharmacologically acceptable salts, pharmaceutical compositions comprising them have hitherto not been described. The compound which is the object of the present invention has not been described either as anti-inflammatory substance or as an inhibitor of eosinophilic accumulation in inflammation tissues.

In one aspect, the present disclosure relates to:

a) compounds represented by Formula **I:**

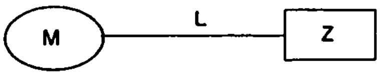

wherein **M** represents a macrolide subunit with substructure **VIII:**

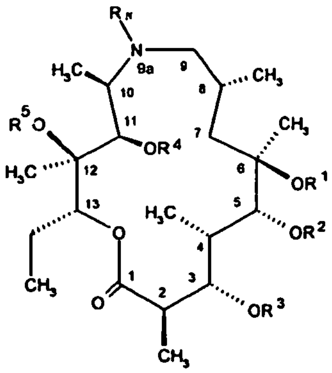

**VIII**

wherein
$R^1$, $R^2$, $R^3$, $R^4$ and $R^5$ are, independently of each other, hydrogen or groups such as $C_1$-$C_4$ alkyl (preferably methyl), alkanoyl (preferably acetyl), alkoxycarbonyl (preferably methoxycarbonyl or *tert*-butoxycarbonyl), arylmethoxycarbonyl (preferably benzyloxycarbonyl), aroyl (preferably benzoyl), arylalkyl (preferably benzyl), alkylsilyl (preferably trimethylsilyl), alkylsilylalkoxyalkyl (preferably trimethylsilylethoxymethyl) or a covalent bond with $X^1$ of chain **L** of formula **IX** or **XIII;** or $R^4$ is a group that can combine with $R^5$ to form a cyclic carbonate or carbamate, or with $>NR_N$ forms a cyclic carbamate.

In another aspect $R^1$, $R^2$, $R^3$, $R^4$ and $R^5$ are independantly chosen from the group consisting of $C_1$-$C_4$ alkyl and hydrogen.

In another aspect $R^1$, $R^2$, $R^3$, $R^4$ and $R^5$ are independantly chosen from the group consisting of methyl and hydrogen.

In another aspect $R^1$, $R^2$, $R^3$, $R^4$ and $R^5$ are hydrogen.

In another aspect $R^4$ is group that can combine with $R^5$ to form a cyclic carbonate or carbamate or $R^4$ is a group that can combine with $>NR_N$ to form a cyclic carbamate.

In another aspect $R^4$ represents a covalent bond with $X^1$ of chain **L** of formula **IX.**

$R_N$ represents hydrogen, $C_1$-$C_4$ alkyl group or the covalent bond with $X^1$ of chain **L** of formula **IX** or **XIII.**

**L** represents a linker chain with substructure **IX** or **XIII:**

$$-X^1-(CH_2)_m-Q-(CH_2)_n-X^2- \quad \textbf{IX}$$

$$-X^1-(CH_2)_m-V-(CH_2)_p-Q-(CH_2)_n-X^2- \quad \textbf{XIII}$$

wherein
$X^1$ is selected from: $-CH_2-$, $-CH_2-NH-$, -C(O)-, -OC(O)-, =N-O-, -C(O)NH- or -OC(O)NH-;
$X^2$ is selected from: -NH-, $-CH_2-$, -NHC(O)-, -C(=O), -OC(O)-, -C(=O)O-, or -C(O)NH-;
Q is -NH- or $-CH_2-$;
wherein each $-CH_2-$ or NH- group are optionally substituted by $C_1$-$C_7$-alkyl, $C_2$-$C_7$-alkenyl, $C_2$-$C_7$-alkynyl, $C(O)R^x$, $C(O)OR^x$, $C(O)NHR^x$, $CH_2C(O)OR^x$, wherein $R^x$ may be $C_1$-$C_7$-alkyl, aryl or heteroaryl;
V is -NH- or -NH-C(O)-;
the symbols m, n and p are independently zero or a whole number from 1 to 12
with the proviso that if Q=NH; n cannot be zero.

This definition of the linking group is preferred not only for conjugates of nonsteroids and macrolides of Formula **VIII** but for any conjugate within Formula **I.** Other linking groups can be used as long as they provide the necessary spacer and can serve to link one subunit of the Formula **I** with the other, as is well-known in the art. For example at U.S. Patent 6,297,260, claim 1 and the specific list of NSAIDs contained therein.

**Z** represents a nonsteroidal subunit derived from nonsteroidal anti-inflammatory drugs (NSAID) or a steroid subunit preferably a steroid of substructure **X:**

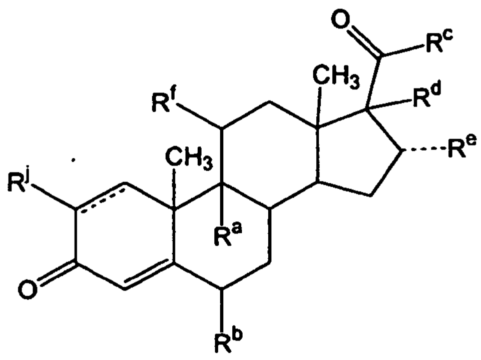

**X**

wherein

$R^a$, $R^b$, independently, are hydrogen, methyl or halogen;

$R^f$ is hydrogen, hydroxyl group or halogen (preferably chlorine) or forms a C=O (carbonyl) group with the carbon atom to which it is linked;

$R^c$ is hydroxy; $C_1$-$C_4$ alkyl (preferably methyl); $C_1$-$C_4$ alkoxy (preferably methoxy); $C_1$-$C_4$alkylhydroxy (preferably $CH_2OH$) NH-$C_1$-$C_4$ alkyl (preferably $NHCH_3$); $CH_2OC(O)C_1$-$C_4$alkyl (preferably $CH_2OC(O)CH_3$); $XC(O)N(R^1R^2)$ wherein X is S or O, $R^1$ and $R^2$ are independently $C_1$-$C_6$ alkyl or $R^1$ and $R^2$ together are $C_1$-$C_6$ alkylene; or $R^c$ is $SCH_2Y$ or $CH_2Y$ wherein Y is halogen (preferably chlorine or fluorine) or $R^c$ is the covalent link with $X^2$ of chain L provided that chain L is linked to $R^4$ of macrolide subunit of formula **VIII;**

$R^d$ is the covalent link with $X^2$ of chain **L,** hydrogen, hydroxy, methyl or $C_1$-$C_4$ alkoxy (preferably methoxy or n-propoxy) or together with $R^e$ and the pertaining C-atoms represent 1,3-dioxolane ring which can be additionally alkyl or alkenyl mono or di-substituted (preferably 2,2-dimethyl or 2-monopropyl or trans-propenyl ring);

$R^e$ is hydrogen, hydroxy, methyl or $C_1$-$C_4$ alkoxy (preferably methoxy or n-propoxy) or together with the $R^d$ and pertaining C-atoms represent 1,3-dioxolane ring which can be additionally alkyl or alkenyl mono or di-substituted (preferably 2,2-dimethyl or 2-monopropyl or trans-propenyl ring).

In another aspect $R^d$ is preferably the covalent link with $X^2$ of chain **L** ; $R^j$ is hydrogen or halogen (preferably chlorine).

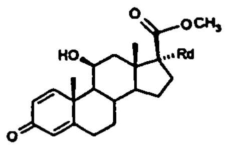
.

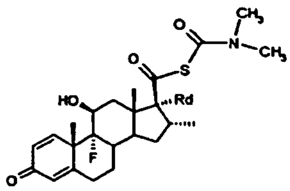
.

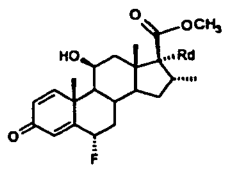
.

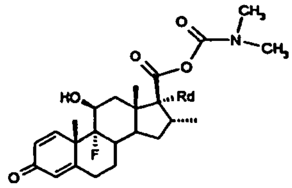
.

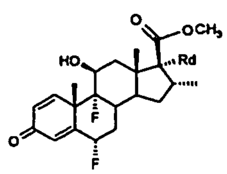
.

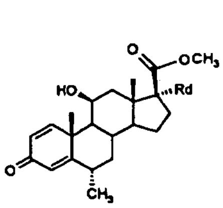
.

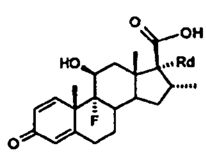
.

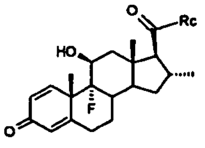,

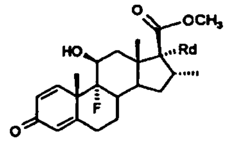,

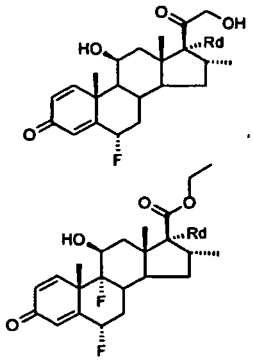,

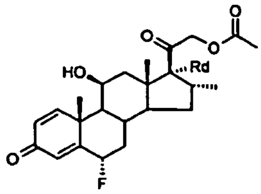,

.

and

In another aspect, the present disclosure relates to processes for preparation of the foregoing compounds and to intermediates which may be used in such preparation.

In a third aspect, the present disclosure relates to combinations of one or more of the foregoing compounds in quantities sufficient for suppression of inflammatory processes; (e.g. two or more NSAID conjugates of the disclosure, two or more steroid conjugates of the invention and disclosure, two or more compounds of the invention and disclosure with at least one being an NSAID conjugate of the disclosure and at least one being a steroid conjugate of the invention and disclosure.) These combinations offer more pronounced antiinflammatory activity if needed to treat inflammatory disease and conditions.

In yet another aspect of the invention and disclosure is pharmaceutical composition comprising a compound of the invention and disclosure and pharmaceutically acceptable salts or solvates thereof including pharmaceutically acceptable diluent or carrier are contemplated. Examples include but are not limited to carboxymethylcellulose and salts thereof, polyacrylic acid and salts thereof, carboxyvinyl polymers and salts thereof, alginic acid and salts thereof, propylene glycol alginate, chitosan, hydroxypropylcellulose, hydroxypropylmethycellulose, hydroxyethylcellulose, ethylcellulose, methycellulose, polyvinyl alcohol, polyvinyl pyrolidone, N-vinylacetamide polymer, polyvinyl methacrylate, polyethylene glycol, pluronic, gelatin, methyl vinyl ether-maleic anhydride copolymer, starch, soluble starch croscaremlose, pullulan and a copolymer of methyl acrylate and 2-ethylhexyl acrylate lecithin, lecithin derivative, propylene glycol fatty acid esters, glycerin fatty acid esters, sorbitan fatty acid esters, polyoxyethylene sorbitan fatty acid esters, polyethylene glycol fatty acid esters polyoxyethylene hydrated caster oil, polyoxyethylene alkyl ethers, and pluronic. Appropriate buffer system if diluent is used is in pH range of 4 to 8, together with low molecular weight alcohols like thanol and isopropanol. The use of preservatives and masking agents is suitable.

In yet another aspect of the invention and disclosure is use of the compound of the invention and disclosure in the manufacture of a medicament for the treatment of inflamatory diseases, disorders, and conditions characterized by or associated with an undesirable inflammatory immune response and all diseases and conditions induced by or associated with an excessive secretion of TNF-$\alpha$ and IL-1.

In yet another aspect of the invention and disclosure is use of the compound of the invention and disclosure in the manufacture of a medicament for the treatment of inflammatory conditions and immune or anaphylactic disorders associated with infiltration of leukocytes into inflamed tissue.

In yet another aspect of the invention and disclosure inflammatory conditions and immune disorders to be treated by the compounds of the invention and disclosure are chosen from the group consisting of asthma, adult respiratory distress syndrome, bronchitis, cystic fibrosis, rheumatoid arthritis, rheumatoid spondylitis, osteoarthritis, gouty arthritis, uveitis, conjunctivitis, inflammatory bowel conditions, Crohn's disease, ulcerative colitis, distal proctitis, psoriasis, eczema, dermatitis, coronary infarct damage, chronic inflammation, endotoxin shock, and smooth muscle proliferation disorders.

In yet another aspect of the invention and disclosure inflammatory conditions and immune disorders to be

treated by the compounds of the invention and disclosure are chosen from the group consisting of asthma, adult respiratory distress syndrome, chronic obstructive pulmonary disorder (COPD) inflammatory bowel conditions, Crohn's disease, bronchitis, and cystic fibrosis.

In yet another aspect of the invention and disclosure is use of the compound of the invention and disclosure in the manufacture of a medicament for the treatment of inflammatory diseases, disorders and conditions characterized by or associated by excessive unregulated production of cytokines or inflamatory mediators.

Symbols **M, L** and **Z** represent three different subunits of compounds of Formula **I.** The symbol M represents the macrolide subunit, and the symbol **Z** represents the steroid or nonsteroidal subunit linked through the chain **L** with the macrolide subunit **M.**

In Formula **I, Z** can represent a nonsteroidal anti-inflammatory subunit, i.e., a moiety of a nonsteroidal antiinflammatory drug (NSAID). Suitable NSAIDs include, but are not limited to, those which inhibit cyclooxygenase, the enzyme responsible for the biosyntheses of the prostaglandins and certain autocoid inhibitors, including inhibitors of the various isoenzymes of cyclooxygenase (including, but not limited to, cyclooxygenase-1 and -2), and as inhibitors of both cyclooxygenase and lipoxygenase relates to nonsteroidal anti-inflammatory drug (NSAID), such as the commercially available NSAIDs aceclofenac, acemetacin, acetaminophen, acetaminosalol, acetyl-salicylic acid, acetyl-salicylic-2-amino-4-picoline-acid, 5-aminoacetylsalicylic acid, alclofenac, aminoprofen, amfenac, ampyrone, ampiroxicam, anileridine, bendazac, benoxaprofen, bermoprofen, α-bisabolol, bromfenac, 5-bromosalicylic acid acetate, bromosaligenin, bucloxic acid, butibufen, carprofen, celecoxib, cromoglycate, cinmetacin, clidanac, clopirac, sodium diclofenac, diflunisal, ditazole, droxicam, enfenamic acid, etodolac, etofenamate, felbinac, fenbufen, fenclozic acid, fendosal, fenoprofen, fentiazac, fepradinol, flufenac, flufenamic acid, flunixin, flunoxaprofen, flurbiprofen, glucametacin, glycol salicylate, ibufenac, ibuprofen, ibuproxam, indomethacin, indoprofen, isofezolac, isoxepac, isoxicam, ketoprofen, ketorolac, lornoxicam, loxoprofen, meclofenamic acid, mefenamic acid, meloxicam, mesalamine, metiazinic acid, mofezolac, montelukast, mycophenolic acid, nabumetone, naproxen, niflumic acid, nimesulide, olsalazine, oxaceprol, oxaprozin, oxyphenbutazone, paracetamol, parsalmide, perisoxal, phenyl-acetyl-salicylate, phenylbutazone, phenylsalicylate, pirazolac, piroxicam, pirprofen, pranoprofen, protizinic acid, reserveratol, salacetamide, salicylamide, salicylamide-O-acetic acid, salicylsulphuric acid, salicin, salicylamide, salsalate, sulindac, suprofen, suxibuzone, tamoxifen, tenoxicam, theophylline, tiaprofenic acid, tiaramide, ticlopidine, tinoridine, tolfenamic acid, tolmetin, tropesin, xenbucin, ximoprofen, zaltoprofen, zomepirac, tomoxiprole, zafirlukast and cyclosporine. Additional NSAID genera and particular NSAID compounds are disclosed in U.S. Patent 6,297,260 (especially in the generic formulas of its claim 1 and the recitation of specific list of NSAID's contained therein and in claim 3), and thiazulidene NSAIDs disclosed in International Patent Application WO 01/87890. Preferred are flufenamic acid, flunixin and celecoxib. In certain embodiments, the NSAID subunit is neither acetyl salicylic acid nor mycophenolic acid.

In formula **I, Z** may also represent a steroid subunit including, but not limited to, corticosteroids (such as glucocorticoids and mineralocorticoids) and androgens. Non-limiting examples of corticosteroids include cortisol, cortisone, clobetasol, hydrocortisone, fludrocortisone, fludroxycortide, flumetasone, flunisolide, fluocinolone, fluocinonide, fluocortolone, fluorometholone, prednisone, prednisolone, 6-alpha-methylprednisolone, triamcinolone, alclometasone, beclometasone, betamethasone, budesonide, dexamethasone, amcinonide, cortivazol, desonide, desoximethasone diflucortolone, difluprednate, fluclorolone and dichlorisone, fluperinidene, fluticasone, halcinonide, meprednisone, methylprednisolone, paramethasone, prednazoline, prednylidene, tixocortol, triamcinolone, and acid derivatives thereof, e.g., acetate, propionate, dipropionate, valerate, phosphate, isonicotinate, metasulfobenzoate, tebutate, and hemisuccinate).

Unless stated otherwise, the following terms have the meanings ascribed to them below.

"Halogen" means a halogen atom which may preferably be: fluorine, chlorine or bromine (the most preferably fluorine or chlorine).

"Alkyl" means a linear or branched saturated monovalent hydrocarbon radical of one to ten carbon atoms, more preferably one to six carbon atoms The preferred straight-chain or branched-chain alkyls include methyl, ethyl, propyl, iso-propyl, butyl, sec-butyl and tert-butyl. $C_1$-$C_4$ alkyl is prefered. Methyl is most preferred. Alkyl groups may be substituted with one up to five substituents including halogen (preferably fluorine or chlorine), hydroxy, alkoxy (preferably methoxy or ethoxy), acyl, acylamino cyano, amino, N-($C_1$-$C_4$)alkylamino (preferably N-methylamino or N-ethylamino), N,N-di ($C_1$-$C_4$-alkyl)amino (preferably dimethylamino or diethylamino), aryl (preferably phenyl) or heteroaryl, thiocarbonylamino, acyloxy, amino, amidino, alkylamidino, thioamidino, aminoacyl, aminocarbonylamino, aminothiocarbonylamino, aminocarbonyloxy, aryl, heteroaryl, aryloxy, aryloxyaryl, nitro, carboxyl, carboxylalkyl, carboxyl-substituted alkyl, carboxylcycloalkyl, carboxyl-substituted cycloalkyl, carboxylaryl, carboxyl-substituted aryl, carboxylheteroaryl, carboxyl-substituted heteroaryl, carboxylheterocyclic, carboxyl-substituted heterocyclic, cycloalkyl, cycloalkoxy, heteroaryloxy, heterocyclyloxy, and oxycarbonylamino. Such substituted alkyl groups are within the present definition of "alkyl." The present definition of alkyl carries over to other groups having an alkyl moiety such as alkoxy or alkanoyl.

"Alkenyl" means a linear or branched monovalent hydrocarbon radical of two to ten and preferably two to six carbon atoms which has at least one double carbon-carbon bond. Alkenyl groups may be substituted with the same groups as alkyl and such optionally substituted alkenyl groups are encompassed within the term "alkenyl". Ethenyl,

propenyl, butenyl and cyclohexenyl are preferred.

"Alkynyl" means a linear or branched monovalent hydrocarbon radical, having a straight-chain or a branched-chain of two to ten, and preferably two to six carbon atoms and containing at least one and preferably no more than three triple carbon-carbon bonds. Alkynyl groups can be substituted with the same groups as alkyl, and the substituted groups are within the present definition of alkynyl. Ethynyl, propynyl and butynyl groups are preferred.

"Cycloalkyl" means a cyclic group having 3-8 carbon atoms having a single ring optionally fused to an aryl or heteroaryl group. The cycloalkyl groups can be substituted as specified for "aryl" below, and the substituted cycloalkyl groups are within the present definition of "cycloalkyl". Preferred cycloalkyls are cyclopentyl and cyclohexyl.

"Aryl" means an unsaturated aromatic carbocyclic group having 6-14 carbon atoms having a single ring such as phenyl or multiple fused rings such as naphthyl. Aryl may optionally be further fused to an aliphatic or aryl group or can be substituted with one or more substituents such as halogen (fluorine, chlorine and/or bromine), hydroxy, $C_1$-$C_7$ alkyl, $C_1$-$C_7$ alkoxy or aryloxy, $C_1$-$C_7$ alkylthio or arylthio, alkylsulfonyl, cyano or primary or nonprimary amino.

"Heteroaryl" means a monocyclic or a bicyclic aromatic hydrocarbon ring having from 2 to 10 carbon atoms and from 1 to 4 heteroatoms, such as O, S or N. The heteroaryl ring may optionally be fused to another heteroaryl, aryl or aliphatic cyclic group. Examples of this type are furan, thiophene, imidazole, indole, pyridine, oxazole, thiazole, pyrrole, pyrazole, tetrazole, pyrimidine, pyrazine and triazine, with furan, pyrrole, pyridine and indole being preferred. The term includes groups that are substituted with the same substituents as specified for aryl above.

"Heterocyclic" means a saturated or unsaturated group having a single or multiple rings and from 1 to 10 carbon atoms and from 1-4 heteroatoms selected from nitrogen, sulfur or oxygen, wherein in a fused ring system the other ring or rings can be aryl or heteroaryl. Heterocyclic groups can be substituted as specified for alkyl groups and the thus substituted heterocyclic groups are within the present definition.

When $R^c$ represents a covalent bond, the nonsteroidal or steroid subunit **Z** is linked via $R^c$ with the chain **L** to the R4 of macrolide subunit **M.**

When $R^d$ represents a covalent bond, the nonsteroidal or steroid subunit **Z** is linked via $R^d$ with the chain **L** to the macrolide subunit **M.**

When $R_N$ represents a covalent bond, the macrolide subunit **M** is linked via $R_N$ with the chain **L** to the nonsteroidal or steroid subunit **Z.**

When $R_4$ represents a covalent bond, the macrolide subunit **M** is linked via $R_4$ with the chain **L** to the nonsteroidal or steroid subunit **Z.**

In the preparation of the compounds represented by Formula **I** of the specified pharmacological activity, in the present disclosure certain new compounds were prepared as intermediates in the preparation of pharmacologically active compounds. The present disclosure also relates to such intermediates.

The term "salts" can include acid addition salts or addition salts of free bases. Examples of acids which may be employed to form pharmaceutically acceptable acid addition salts include but are not limited to salts derived from nontoxic inorganic acids such as nitric, phosphoric, sulfuric, or hydrobromic, hydroiodic, hydrofluoric, phosphorous, as well as salts derived from nontoxic organic acids such as aliphatic mono- and dicarboxylic acids, phenyl-substituted alkanoic acids, hydroxyl alkanoic acids, alkanedioic acids, aromatic acids, aliphatic and aromatic sulfonic acids, and acetic, maleic, succinic, or citric acids. Non-limiting examples of such salts include napadisylate, besylate, sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate (see, for example, Berge S. M. et al. "Pharmaceutical Salts," J. of Pharma. Sci., 1977,66,1).

The acid addition salts of said basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention and disclosure.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine.

The base addition salts of said acidic compounds are prepared by contacting the free acid form with a sufficient amount of the desired base to produce the salt in the conventional manner. The free acid form may be regenerated by contacting the salt form with an acid and isolating the free acid in the conventional manner.

The phrase "pharmaceutically acceptable", as used in connection with compositions of the invention and disclosure, refers to molecular entities and other ingredients of such compositions that are physiologically tolerable and do

not typically produce untoward reactions when administered to a mammal (e.g., human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

The term "carrier" applied to pharmaceutical compositions of the invention and disclosure refers to a diluent, excipient, or vehicle with which an active compound is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. However, aqueous solutions are preferred. Suitable pharmaceutical carriers are described in "Remington's Pharmaceutical Sciences" by E.W. Martin, 18th Edition. Particularly preferred for the present invention and disclosure are carriers suitable for immediate-release, i.e., release of most or all of the active ingredient over a short period of time, such as 60 minutes or less, and make rapid absorption of the drug possible.

The present invention and disclosure also encompasses solvates (preferably hydrates) formed by the compounds represented by Formula **I** or their salts.

The present disclosure also relates to all possible tautomeric forms which can be formed by individual compounds of Formula **I.**

The present disclosure also encompasses prodrugs of Formula **I** compounds, i.e., compounds which release an active parent drug according to Formula **(I)** in vivo when administered to a mammalian subject. Prodrugs of a compound of Formula **I** are prepared by modifying functional groups present in the compound of Formula **I in** such a way that the modifications may be cleaved in vivo to release the parent compound. Prodrugs include compounds of Formula **I** wherein a hydroxy, amino, or carboxy group of a Formula **I** compound is bonded to any group that may be cleaved in vivo to regenerate the free hydroxyl, amino or carboxy group, respectively. Examples of prodrugs include, but are not limited to esters (e.g., acetate, formate, and benzoate derivatives) of compounds of Formula **I.**

The compounds of Formula **I** have one or more chirality centers and, depending on the nature of individual substituents, they can also have geometrical isomers. Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has a chiral center, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomer respectively). A chiral compound can exist as either an individual enantiomer or as a mixture of enantiomers. A mixture containing equal proportions of the enantiomers is called a "racemic mixture". The present disclosure encompasses all individual isomers of compounds of Formula **I.** The description or naming of a particular compound in the specification and claims is intended to include both individual enantiomers and mixtures, racemic or otherwise, thereof. Methods for the determination of stereochemistry and the separation of stereoisomers are well-known in the art.

A "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the present application includes both one and more than one such excipient.

"Treating" or "treatment" of a state, disorder or condition includes:

(1) preventing or delaying the appearance of clinical symptoms of the state, disorder or condition developing in a mammal that may be afflicted with or predisposed to the state, disorder or condition but does not yet experience or display clinical or subclinical symptoms of the state, disorder or condition,
(2) inhibiting the state, disorder or condition, i.e., arresting or reducing the development of the disease or at least one clinical or subclinical symptom thereof, or
(3) relieving the disease, i.e., causing regression of the state, disorder or condition or at least one of its clinical or subclinical symptoms.

The benefit to a subject to be treated is either statistically significant or at least perceptible to the patient or to the physician
A "therapeutically effective amount" means the amount of a compound that, when administered to a mammal for treating a state, disorder or condition, is sufficient to effect such treatment. The "therapeutically effective amount" will vary depending on the compound, the disease and its severity and the age, weight, physical condition and responsiveness of the mammal to be treated.

The four classic symptoms of acute inflammation are redness, elevated temperature, swelling, and pain in the affected area, and loss of function of the affected organ.

Symptoms and signs of inflammation associated with specific conditions include:

- rheumatoid arthritis- pain, swelling, warmth and tenderness of the involved joints; generalized and morning stiffness;
- insulin-dependent diabetes mellitus- insulitis; this condition can lead to a variety of complications with an inflammatory component, including: retinopathy, neuropathy, nephropathy; coronary artery disease, peripheral vascular disease, and cerebrovascular disease;
- autoimmune thyroiditis- weakness, constipation, shortness of breath, puffiness of the face, hands and feet, peripheral edema, bradycardia;
- multiple sclerosis- spasticity, blurry vision, vertigo, limb weakness, paresthesias;
- uveoretinitis- decreased night vision, loss of peripheral vision;
- lupus erythematosus-joint pain, rash, photosensitivity, fever, muscle pain, puffiness of the hands and feet, abnormal urinalysis (hematuria, cylinduria, proteinuria), glomerulonephritis, cognitive dysfunction, vessel thrombosis, pericarditis;
- scleroderma- Raynaud's disease; swelling of the hands, arms, legs and face; skin thickening; pain, swelling and stiffness of the fingers and knees, gastrointestinal dysfunction, restrictive lung disease; pericarditis,; renal failure;
- other arthritic conditions having an inflammatory component such as rheumatoid spondylitis, osteoarthritis, septic arthritis and polyarthritis- fever, pain, swelling, tenderness;
- other inflammatory brain disorders, such as meningitis, Alzheimer's disease, AIDS dementia encephalitis- photophobia, cognitive dysfunction, memory loss;
- other inflammatory eye inflammations, such as retinitis- decreased visual acuity;
- inflammatory skin disorders, such as , eczema, other dermatites (e.g., atopic, contact), psoriasis, bums induced by UV radiation (sun rays and similar UV sources)-erythema, pain, scaling, swelling, tenderness;
- inflammatory bowel disease, such as Crohn's disease, ulcerative colitis- pain, diarrhea, constipation, rectal bleeding, fever, arthritis;
- asthma- shortness of breath, wheezing;
- other allergy disorders, such as allergic rhinitis- sneezing, itching, runny nose
- conditions associated with acute trauma such as cerebral injury following stroke-sensory loss, motor loss, cognitive loss;
- heart tissue injury due to myocardial ischemia- pain, shortness of breath;
- lung injury such as that which occurs in adult respiratory distress syndrome- shortness of breath, hyperventilation, decreased oxygenation, pulmonary infiltrates;
- inflammation accompanying infection, such as sepsis, septic shock, toxic shock syndrome- fever, respiratory failure, tachycardia, hypotension, leukocytosis;
- other inflammatory conditions associated with particular organs or tissues, such as
  nephritis (e.g., glomerulonephritis)-oliguria, abnormal urinalysis;
  inflamed appendix- fever, pain, tenderness, leukocytosis;
  gout- pain, tenderness, swelling and erythema of the involved joint, elevated serum and/or urinary uric acid;
  inflamed gall bladder- abdominal pain and tenderness, fever, nausea, leukocytosis;
  chronic obstructive pulmonary disorder (COPD), shortness of breath, wheezing;
  congestive heart failure- shortness of breath, rales, peripheral edema;
  Type II diabetes- end organ complications including cardiovascular, ocular, renal, and peripheral vascular disease
  lung fibrosis- hyperventilation, shortness of breath, decreased oxygenation;
  vascular disease, such as atherosclerosis and restenosis- pain, loss of sensation, diminished pulses, loss of function
  and alloimmunity leading to transplant rejection-pain, tenderness, fever.

Subclinical symptoms include without limitation diagnostic markers for inflammation the appearance of which may precede the manifestation of clinical symptoms. One class of subclinical symptoms is immunological symptoms, such as the invasion or accumulation in an organ or tissue of proinflammatory lymphoid cells or the presence locally or peripherally of activated pro-inflammatory lymphoid cells recognizing a pathogen or an antigen specific to the organ or tissue. Activation of lymphoid cells can be measured by techniques known in the art.
"Delivering" a therapeutically effective amount of an active ingredient to a particular location within a host means causing a therapeutically effective blood concentration of the active ingredient at the particular location. This can be accomplished, e.g., by local or by systemic administration of the active ingredient to the host.
The term host or subject in need thereof as used herein refers to a mammal preferably a human.
The term leaving group refers to a chemical group which is capable of being displaced by a nucleophile. Examples of such groups include but are not limited to halogen, mesylate, tosylate and ester groups.

**Methods of Preparation**

The present disclosure relates to a method for the preparation of compounds within Formula I comprising:

a) for the compounds of Formula It wherein $X^2$ is -NH-
a reaction of the steroid or nonsteroidal subunit of the substructure **V**:

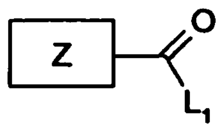

**XI**

(wherein $L_1$ represents a leaving group such as hydroxy)
and the amino group of the macrolide subunit of the substructure **VIa:**

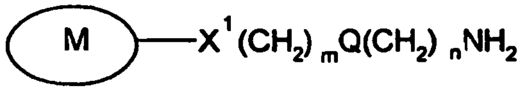

**XII**

Steroid or nonsteroidal subunits of the substructure **XI** are either commercially available products or have been obtained, like the starting macrolides subunits of the substructure **XII** by methods for preparation of analogous compounds described in patent applications HR 20010018; WO 02/055531; WO 04/005309; and WO 04/005310. The reaction is generally performed with acid derivatives which have the ability to activate the carboxylic acid group of steroidal anti-inflammatory subunit, such as halogenides, mixed anhydrides and especially carbodiimides (such as 1-(3-dimethylaminopropyl)-3-ethyl-carbodiimide (EDC)) and benzotriazoles. The reaction proceeds in the presence of a base, such as an organic base (e.g., triethylamine), at room temperature under an inert atmosphere such as nitrogen or argon. The reaction may require several hours to several days to come to completion.
b) Compounds represented by Formula **I**, where $X^1$ is -C(O)NH-, Q is $-CH_2-$ or NH- and $X^2$ is -NH- or NHC(O)-, can be prepared by reacting a macrolide subunit and a derivatized steroid or nonsteroidal subunit having a free amino group as shown below.

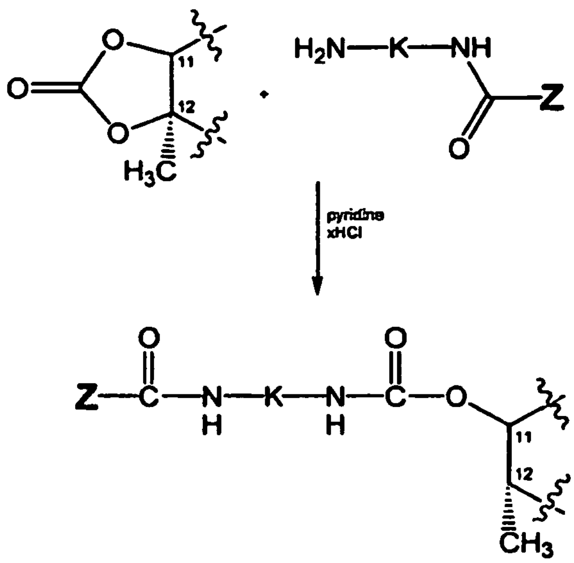

c) Compounds represented by Formula **I**, where $X^1$ is -C(O)NH-, Q is $-CH_2-$ or -NH- and $X^2$ is -NH- or NHC(O)-, can be prepared by reacting a macrolide subunit and a steroid or nonsteroidal subunit having a free carboxylic acid group as shown below.

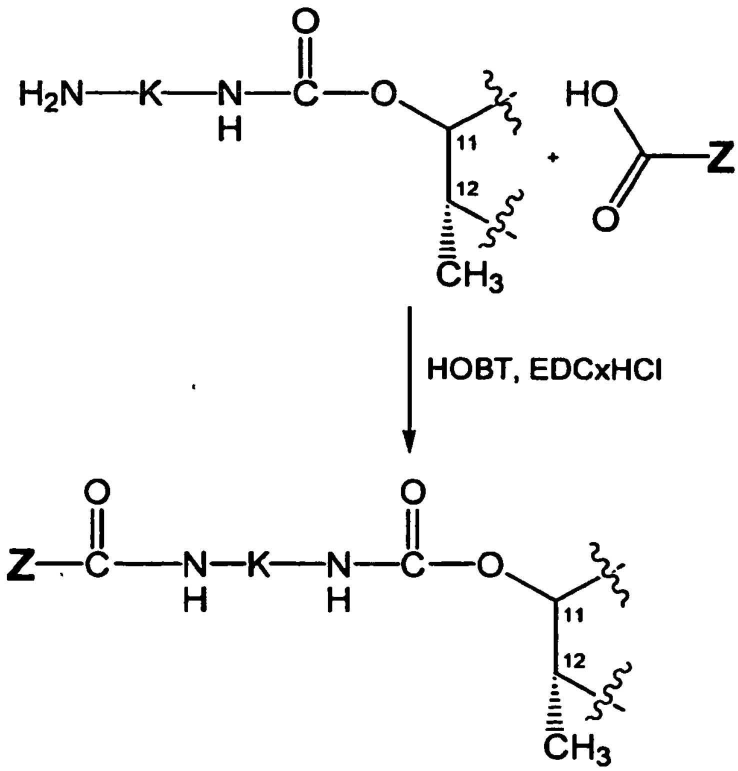


The steroid subunit may be linked to the macrolide through the 21 hydroxy group in steroids that have such a group. Begining with a 21-hydroxy steroid cyclic ketal is reacted with an appropriate carboxylic acid halide or an anhydride, preferably in a solvent such as methylene chloride in the presence of a tertiary amine base or pyridine at a reduced temperature (-50° C-100° C). The intermediate so produced is reacted with $H_2N$-L-M to form compounds of Formula **I.**

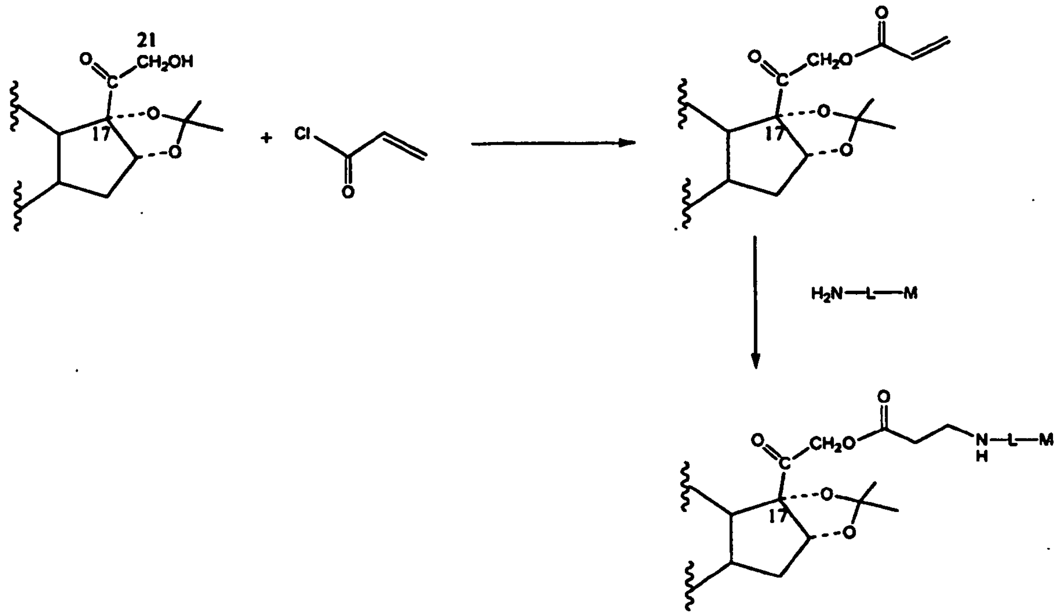


The steroid subunit may also be linked to the macrolide through the 17 position on the steroid subunit. One method for preparing such a compound is as follows:

As illustrated by the synthetic schemes above and below there are two synthetic pathway alternatives: one derivatives the carboxylic acid group at $C_{17}$ to form C(O)-$R^c$ prior to coupling with the linker-macrolide partion, and the other derivatives the same carboxylic group only subsequesnt to such coupling.

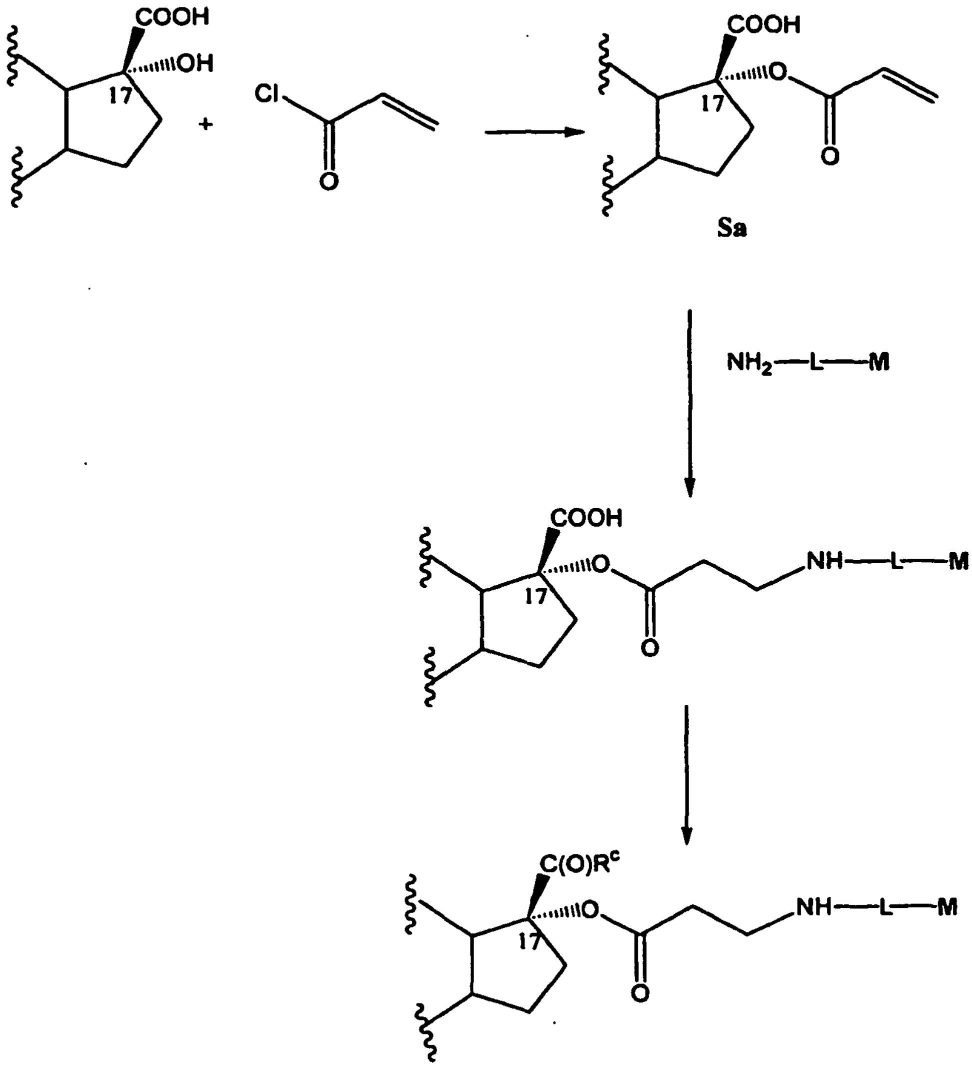


For example, when L is -K-NH- (wherein K is the portion of the L molecule attached to the macrolide) the compound of Formula **I** can be formed by derivatizing an NH group on the macrolide ring to a terminal -N-K-$NH_2$ group and reacting the derivatized macrolide with a steroid anti-inflammatory subunit represented by Formula **Sa:**

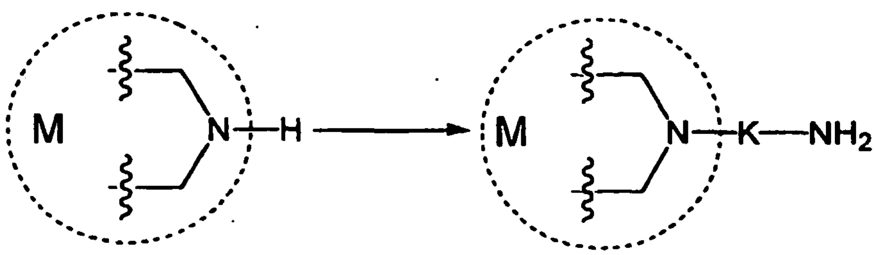

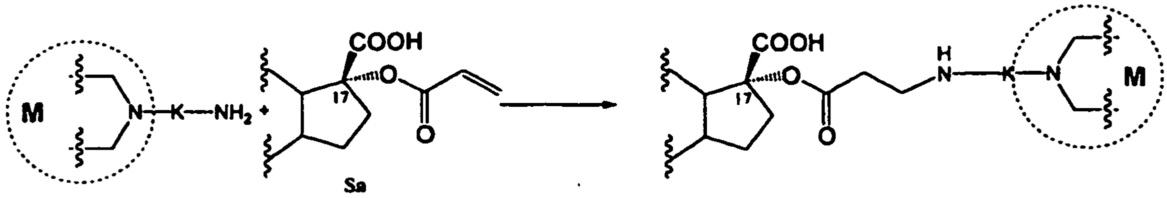

Compounds represented by Formula **I**, where $X^2$ is -NH-, can be prepared by reacting a macrolide subunit and a steroid subunit having a -C=C- bond as shown below.

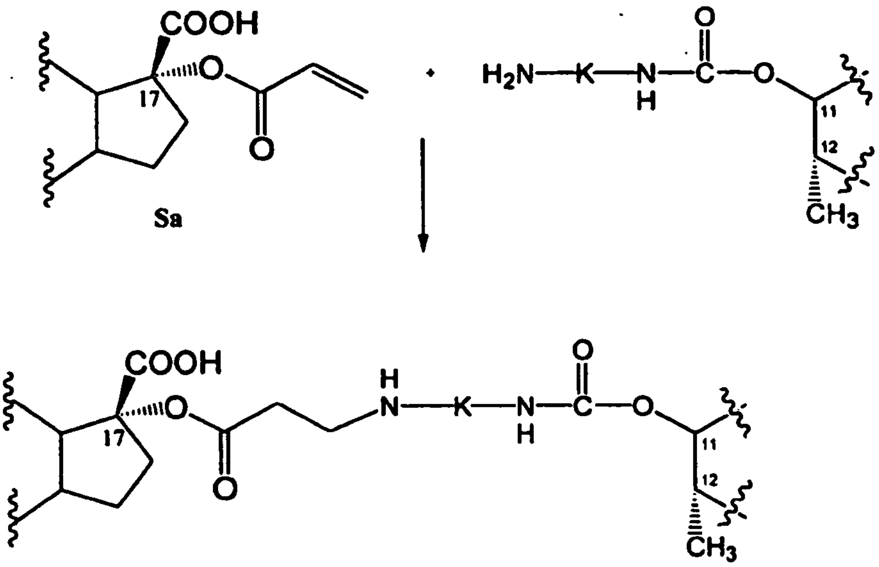

The carboxylic acid group at the 17 position of the starting steroid subunit may be modified prior to the reaction with $NH_2$-L-**M**.

The carboxylic acid group at the 17 position of the starting steroid subunit can also be protected prior to the reaction with $NH_2$-L-**M** and deprotected after the reaction with $NH_2$-L-M or the esterification step.

The non-steroidal anti-inflammatory subunit D may contain a -C(O)$L^1$ group (such as a free carboxylic acid group) or be derivatized by methods known in the art.

## Scheme I

1. Pyr
2. $NEt_3$, 4-PP, $CH_2Cl_2$

NSAID-OH + SUCCINIC ANHYDRIDE → NSAID-OC(O)$CH_2CH_2$COOH

According to Scheme I, NSAID compounds having a hydroxyl group may alternatively be derivatized by the action of succinic anhydride in the presence of pyridine followed by reaction of the intermediate so produced with triethylamine, 4-pyrrolopyridine in methylene chloride to produce NSAID having free carboxylic acid group (Huang C.M. et al.

Chem.&Bio/. 2000, 7, 453-461, Mess S. et al. Bioorg.&Med Chem. 2001, 9, 1279-1291). The NSAID derivatives so produced may be coupled to a linker macrolide compound such as formula VIa.

Scheme II

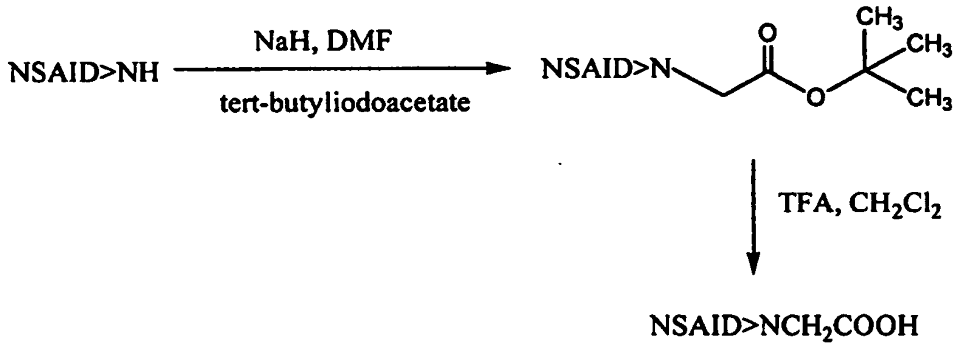


According to Scheme II, NSAID compounds having an amino group may alternatively be derivatized by the action of sodium hydride and tert-butyliodoacetate in N,N-dimethylformamide to produce a (butoxy carbonyl derivative of the NSAID which is then reacted with (trifluoracetic acid in methylene chloride to produce NSAID having free carboxylic acid group (Hess S. et al. Bioorg.&Med Chem. 2001, 9, 1279-1291). The NSAID derivatives so produced may be coupled to a linker macrolide compound such as formula VIa.

Scheme III

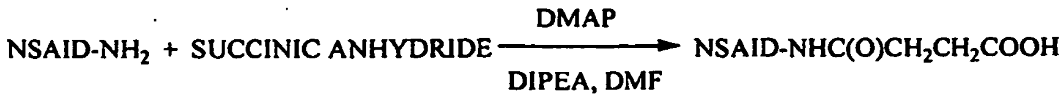


Alternatively NSAID compounds having an amino group may be derivatized according to Scheme III by the action of succinic anhydride in the presence of dimethylaminopyridine, N,N'-diisopropylethylamine in dimethylformamide to produce NSAID having free carboxylic acid group (Pandori M. W. et al. Chem. &Biol. 2002, 9, 567-573). The NSAID derivatives so produced may be coupled to a linker macrolide compound such as formula **VIa.**

Compounds of Formula **I** can generally be obtained so that: one end of the chain **L** is first linked to the macrolide subunit **M,** and then the other end of the chain is linked to the nonsteroidal or steroid subunit **D;** or one a of the chain **L** is first linked to the nonsteroidal or steroid subunit **D** and then the other end of the chain is linked to the macrolide subunit **M,** and, finally, one end of the yet unformed chain is linked to the macrolide subunit **M,** and the other end of the also unformed chain is linked to the nonsteroidal or steroid subunit **D,** and subsequently the ends are chemically linked to form the chain **L.**

To prevent undesirable side-reactions, it is frequently necessary to protect certain groups such as e.g. a hydroxy or amino group. **A** comprehensive discussion of the ways in which such groups may be protected and methods for cleaving the resulting protected derivatives is given by for example T.W. Greene and P.G.M Wuts in Protective Groups in Organic Synthesis 2nd ed., John Wiley & Son, Inc 1991 and by P.J. Kocienski in Protecting Groups, Georg Thieme Verlag 1994. Examples of suitable amino protecting groups include acyl type protecting groups (e.g. formyl, trifluoroacetyl and acetyl), aromatic urethane type protecting groups (e.g. benzyloxycarbonyl (Cbz) and substituted Cbz, and 9-fluorenylmethoxycarbonyl (Fmoc)), aliphatic urethane protecting groups (e.g. t-butyloxycarbonyl (Boc), isopropyloxycarbonyl and cyclohexyloxycarbonyl) and alkyl type protecting groups (e.g. benzyl, trityl and chlorotrityl). Examples of suitable oxygen protecting groups may include for example alkyl silyl groups, such as trimethylsilyl or tert-butyldimethylsilyl; alkyl ethers such as tetrahydropyranyl or tert-butyl; or esters such as acetate. Hydroxy groups may be protected by reaction of for example acetic anhydride, benzoic anhydride or a trialkylsilyl chloride in an aprotic solvent. Examples of aprotic solvents are dichloromethane, N,N-dimethylformamide, dimethylsulfoxide, tetrahydrofuran and the like.

For example, one possibility for the protection of the amino group is t-butyloxycarbonyl (Boc). Deprotection using trifluoroacetic acid (TFA) is described in the examples.

Corresponding protection for amino and alkylamino groups are groups such as alkanoyl (acetyl), alkoxycarbonyl (methoxycarbonyl, etoxycarbonyl or *tert*-butoxycarbonyl), arylmethoxycarbonyl (benzyloxycarbonyl), aroyl (benzoyl) and alkylsilyl group (trimethylsilyl or trimethylsilyletoxymethyl). The conditions for elimination of the protective group depend

on the selection and properties of that group. Thus, for example, acyl groups such as alkanoyl, alkoxycarbonyl and aroyl group can be removed by hydrolysis in the presence of a base (sodium or potassium hydroxide), *tert*-butoxycarbonyl or alkylsilyl (trimethylsilyl) group can be removed with a corresponding acid (for example, hydrochloric, sulphuric, phosphoric or trifluoroacetic acid), while arylmethoxycarbonyl group (benzyloxycarbonyl) can be removed by hydrogenolysis in the presence of a catalyst such as palladium-on-charcoal.

Further, the present invention and disclosure relate to pharmaceutical compositions containing an effective dose of a compound of the present invention or disclosure as well as pharmaceutically acceptable excipients, such as carriers or diluents.

The preparation of the pharmaceutical compositions of the invention and disclosure can include mixing, granulating, tabletting and dissolving the ingredients. Chemical carriers can be in solid or liquid form. Solid carriers can be lactose, sucrose, talc, gelatine, agar, pectin, magnesium stearate, fatty acids without limitation. Liquid carriers can be syrups, oils such as olive, sunflower seed or soybean oils, water, or physiologic saline without limitation. Similarly, carriers may also contain a component for a sustained release of the active component such as glyceryl monostearate or glyceryl distearate. Several forms of pharmaceutical compositions can be prepared. If a solid carrier is used, these forms can include tablets, caplets, solid gelatinous capsules, powders or granules without limitation that can be administered orally. The amount of the solid carrier can vary but mainly it is in the range from 25 mg to 1 g. If a liquid carrier is used, the formulation can be in the form of a syrup, emulsion, soft gelatinous capsules, or sterile injectable liquids, or nonaqueous liquid suspensions topically or systemically, e.g., orally, parenterally, percutaneously, mucosally, e.g., buccally, intranasally, intrarectally and intravaginally. "Parenterally" means by intravenous, intramuscular or subcutaneous route.

The corresponding preparations of the compound of the present invention and disclosure can be used in the prophylaxis as well as in the therapeutic treatment (prevention, delay, inhibition or relief) of several disorders (diseases and other pathological inflammatory conditions) caused by or associated with an abnormal or undesirable (excessive, nonregulated, or dysregulated) inflammatory immune response involving the production of inflammatory cytokines or other inflammation mediators, including without limitation TNF-$\alpha$ and IL-1$\beta$. These disorders include autoimmune diseases such as rheumatoid arthritis, insulin-dependent diabetes mellitus, autoimmune thyroiditis, multiple sclerosis, uveoretinitis, lupus erythematosus, scleroderma; other arthritic conditions having an inflammatory component such as rheumatoid spondylitis, osteoarthritis, septic arthritis and polyarthritis; other inflammatory brain disorders, such as meningitis, Alzheimer's disease, AIDS dementia encephalitis, other inflammatory eye inflammations, such as retinitis; inflammatory skin disorders, such as , eczema, other dermatites (e.g., atopic, contact), psoriasis, bums induced by UV radiation (sun rays and similar UV sources); inflammatory bowel disease, such as Crohn's disease, ulcerative colitis; asthma; other allergy disorders, such as allergic rhinitis; conditions associated with acute trauma such as cerebral injury following stroke, heart tissue injury due to myocardial ischemia, lung injury such as that which occurs in adult respiratory distress syndrome; inflammation accompanying infection, such as sepsis, septic shock, toxic shock syndrome, other inflammatory conditions associated with particular organs or tissues , such as nephritis (e.g., glomerulonephritis), inflamed appendix, gout, inflamed gall bladder, congestive heart failure, Type II diabetes, lung fibrosis, vascular disease, such as atherosclerosis and restenosis; and alloimmunity leading to transplant rejection. The compounds can also be administered by inhalation when application within the respiratory tract is intended. A further object of the present invention and disclosure relates to the preparation of various pharmaceutical forms of the compounds to achieve the optimal bioavailability of the active compound of Formula **I.**

For percutaneous or mucosal external administration, the compound of Formula I can be prepared in a form of an ointment or cream, gel or lotion. Ointments, creams and gels can be formulated using a water or oil base with addition of an appropriate emulsifier or gelling agent Formulation of the present compounds is especially significant for respiratory inhalation, wherein the compound of Formula **I** is to be delivered in the form of an aerosol under pressure. It is preferred to micronize the compound of Formula **I** after it has been homogenised, e.g., in lactose, glucose, higher fatty acids, sodium salt of dioctylsulfosuccinic acid or, most preferably, in carboxymethyl cellulose, in order to achieve a microparticle size of 5 $\mu$m or less for the majority of particles. For the inhalation formulation, the aerosol can be mixed with a gas or a liquid propellant for dispensing the active substance. An inhaler or atomizer or nebulizer may be used. Such devices are known. See, e.g., Newman et al., Thorax, 1985, 40, 61-676; Berenberg M., J. Asthma USA, 1985, 22: 87-92**.** A Bird nebulizer can also be used. See also U.S. Patents 6,402,733; 6,273,086; and 6,228,346.

The compound of the formula **I** and **Ia** for inhalation is preferably formatted in the form of a dry powder with micronized particles, as described herein.

The compound can also be incorporated into a formulation for treating inflammation localized in an organ or tissue, e.g., Crohn's disease, where it can be administered orally or rectally. Formulations for oral administration can incorporate excipients enabling bioavailability of the compound at the site of inflammation. This can be achieved by different combinations of enteric and delayed release formulations. The compound of formula **I** and **Ia** can also be used in the treatment of Crohn's disease and intestinal inflammation disease if the compound is applied in the form of a clyster, for which a suitable formulation can be used, as is well known in the field.

A therapeutically effective amount of the compound of the present invention and disclosure can be determined

by methods known in the art. Since the compound of the present invention and disclosure is more efficiently delivered to the desired site than the corresponding anti-inflammatory steroid or NSAID drug alone, a lesser amount of the compound on a molar basis than of the steroid or NSAID anti-inflammatory drug can be administered while still achieving the same therapeutic effect. Furthermore, since administration of the compound results in fewer side effects than with the corresponding steroid or NSAID anti-inflammatory drug, the steroid or NSAID amount can be increased. Thus, the table below serves only as a guide. A threshold therapeutically effective amount of the compound, a pharmaceutically salt thereof, a solvate thereof, or a prodrug thereof is generally equal to or less than a therapeutically effective amount of the nonsteroidal anti-inflammatory drug on a molar basis. Broad and preferred effective amounts of the compound, a pharmaceutically salt thereof, a solvate thereof, or a prodrug thereof are shown in the table below.

| | Amount of Compound, Pharmaceutically Acceptable Salt Thereof, Solvate Thereof, or Prodrug Thereof | |
|---|---|---|
| | mg/kg body weight/day of the steroid or NSAID (had it been administered alone) | μmol/kg body weight/day of the hybrid or the steroid or NSAID |
| Broad | from about 0.001 to about 1000 | from about 0.004 to about 4000 |
| Preferred | from about 0.01 to about 100 | from about 0.04 to about 400 |
| More Preferred | from about 1 to about 100 | from about 4 to about 400 |
| Most Preferred | from about 3 to about 30 | from about 12 to about 120 |

For example, if the preferred amount range for prednisone is 1-50 mg/day, this corresponds to a range of 2.79 μmol to 139.5 μmol per day. The starting amount range for a hybrid steroid-macrolide conjugate according to the invention and disclosure will be also 2.79 μmol to 139.5 μmol of conjugate per day. This dosage can be fine-tuned in light of the present specification using the ordinary skill in the art.

The efficacy of the present compounds can be assessed by any method for assessing inflammation or anti-inflammatory effect. There are many known methods for this purpose including without limitation use of contrast ultrasound in conjunction with injection of microbubbles, measurement of inflammatory cytokines (such as TNF-α, IL-1, IFN-γ) measurement of activated immune system cells (activated T cells, cytotoxic T cells specifically recognizing the inflamed or transplanted tissue) as well as by observation (reduction of oedema, reduction of erythema, reduction of pruritus or burning sensation, reduction of body temperature, improvement in function of the afflicted organ) as well as any of the methods provided below as well as any of the methods provided below.

The therapeutic effect of compounds of the present invention and disclosure was determined in *in vitro* and *in vivo* experiments such as the following.

The beneficial antiinflammatory effect of the compounds of the present invention and disclosure was determined in the following *in vitro* and *in vivo* experiments:

Formulations for oral administration can be so designed to enable bioavailability of the compound at the site of inflammation in the intestines. This can be achieved by different combinations of delayed release formulations. The compound of Formula **I** and **Ia** can also be used in the treatment of Crohn's disease and intestinal inflammation disease if the compound is applied in the form of an enema, for which a suitable formulation can be used.

The corresponding preparations of the compounds of the present invention and disclosure can be used in the prophylaxis (including without limitation the prevention, delay or inhibition of recurrence of one or more of the clinical or subclinical symptoms discussed and defined in connection with the definitions of "treatment" above. as well as in the therapeutic treatment of several diseases and and pathological inflammatory conditions including: chronic obstructive pulmonary disorder (COPD) asthma, inflammatory nasal diseases such as allergic rhinitis, nasal polyps, intestinal diseases such as Crohn's disease, colitis, intestinal inflammation, ulcerative colitis, dermatological inflammations such as eczema, psoriasis, allergic dermatitis, neurodermatitis, pruritis, conjunctivitis and rheumatoid arthritis.

The biological effect of the compounds of the present invention and disclosure was determined in the following *in vitro* and *in vivo* experiments:

**Assay of Binding to Human Glucocorticoid Receptor**

The gene for the alpha isoform of human glucocorticoid receptor was cloned by reverse polymerase chain reaction. The total RNA was isolated from human peripheral blood lymphocytes according to the instructions of the manufacturer (Qiagen, Milano, Italy), transcripted into cDNA with AMV reverse transcriptase (Roche, Basel, Switzerland) and the gene was multiplied by specific primers 1) 5'ATATGGATCCCTGATGGACTCCAAAGAATCATTAACTCC3' and 2) 5'ATAT-CTCGAGGGCAGTCACTTTTGATGAAACAGAAG3'. The reaction product obtained was cloned into the XhoI/BamHI site of Bluescript KS plasmid (Stratagene, La Jolla, CA USA), subjected to sequencing by the dideoxy fluorescent method with M13 and M13rev primers (Microsynth, Balgach, Switzerland) and then it was cloned into the XhoI/BamHI site of pcDNA3.1 Hygro(+)plazmid (Invitrogen). $1x10^5$ COS-1 cells were seeded onto a 12-well plate (Falcon) in DMEM medium (Life Technologies, Carlsbad, CA USA) with 10% FBS (Biowhitaker) and cultivated to a 70% confluence at 37°C in an atmosphere with 5% $CO_2$ The medium was removed and 1 μg of DNA, 7 μl of PLUS reagent and 2 μl of Lipofectamin (Life Technologies) in 500 μl of DMEM were added per well. The cells were incubated at 37 °C in an atmosphere with 5% $CO_2$ and after 5 hours the same volume of 20% FBS/DMEM was added. After 24 hours, the medium was completely changed. 48 hours after transfection, the test compounds in different concentrations and 24 nM [$^3$H]dexamethazone (Pharmacia, Piscataway, NJ USA) in DMEM medium were added. The cells were incubated for 90 minutes at 37°C in an atmosphere with 5% $CO_2$, washed three times with PBS buffer (Sigma, St. Louis, MO USA) cooled to 4 °C (pH=7,4), and then lysed in Tris buffer (pH=8,0) (Sigma, St. Louis, MO USA) with 0.2% of SDS (Sigma, St. Louis, MO USA). After the addition of UltimaGold XR (Packard BioScience, Groningen, The Netherlands) scintillation liquid, the residual radioactivity was read in a Tricarb (Packard) β-scintillation counter.

Reference compound 7 and compound **Ia** have affinity for the glucocorticoid receptor since in the assay they displace radioactive dexamethasone from the glucocorticoid receptor. Other compounds of the disclosure will demonstrate similar results when tested in this assay.

**Assay of Inhibition of Mouse T-cell Hybridoma 13 Proliferation as a Result of Apoptosis Induction**

Triplicates of test steroid dilution in RPMI medium (Institute of Immunology, Zagreb) with 10% FBS were added to a 96 well plate. To solutions containing compounds at various concentrations, 20000 cells per well were added and incubated overnight at 37 °C in an atmosphere with 5% $CO_2$. Then 1 μCi of [$^3$H]thymidine (Pharmacia, Piscataway, NJ USA) was added and the mixture was incubated for an additional 3 hours. The cells were harvested by applying a vacuum over GF/C filter (Packard). Onto each well, 30 μl of Microscynt O scintillation liquid (Packard) was added and the incorporated radioactivity was measured on a β-scintillation counter (Packard). The specificity of apoptosis induction by glucocorticoids was demonstrated by antagonizing the proliferation inhibition with mifepristone (Sigma, St. Louis, MO USA) a potent glucocorticoid receptor antagonist.

Reference compounds 7 and 9, and compound **Ia** exhibit inhibition of T-cell hybridoma 13 proliferation in the concentrations from 1 μM to 1 nM. Other compounds of the disclosure will demonstrate antiproliferativeactivity where tested in this assay.

**Table Ia** $IC_{50}$ Values in T-cell Hybridoma 13 Assay

| **Compound** | $IC_{50}$ M |
|---|---|
| **7** | $4,64x10^{-7}$ |
| **9** | $3,03x10^{-7}$ |
| **10** | $4,17x10^{-8}$ |
| **13** | $2,87x 10^{-7}$ |
| **14** | $4,55x10^{-7}$ |
| **16** | $1,12x10^{-7}$ |
| **17** | $4,16x10^{-8}$ |
| **18** | $4,74x10^{-7}$ |

$IC_{50}$ values were calculated using GraphPad Prism software.
All compounds having $IC_{50}$ values below 2 μM are considered active.

**Measurement of the inhibition of interleukin 4, interleukin 5 and interferon γ production by concanavalin-A induced murine splenocytes**

Splenocytes were isolated from the spleen of Balb/C mice sacrificed by thiopental injection (Pliva, Zagreb, Croatia). Spleens were chopped and mononuclear cells separated on Histopaque 1083 (Sigma Diagnostics, St. Louis, MO USA Cat. No 1083-1). Into a 96-well plate, compounds diluted in RPMI medium (Institute of Immunology, Zagreb, Croatia) were pipetted with 10% foetal bovine serum (Biowhittaker) and cells (200000 per well) in the same medium, and concanavalin-A stimulator (Sigma 2002-2003 cat. No C5275) at a final concentration of 5 μg/ml were added. The

positive control, in place of the dilution of compounds, consisted of RPMI medium with 10% foetal bovine serum and concanavalin-A in the same concentration of. Cells were incubated for 72 hours at 37 °C, 95% humidity and in an atmosphere with 5% $CO_2$. Until determination of cytokines, the cells were frozen at -70 °C.

Cytokines interleukin 4, interleukin 5 and interferon γ were determined by the specific ELISA method, according to manufacturer's recommendations (R&D).

Inhibition (as percentage) was calculated using the following formula:

$$\%inh = (1 - [\text{concentration of cytokines in sample}]/[\text{concentration of cytokines in positive control}]) * 100$$

Compound **Ia** inhibits the production of cytokines in concentrations from 1 μM to 1 nM.

**Model of Lung Eosinophilia in Mice**

Male Balb/C mice with a body weight of 20-25 g were randomly divided into groups, and sensitised by an i.p. injection of ovalbumin (OVA, Sigma, St. Louis, MO USA) on day zero and day fourteen. On the twentieth day, the mice were subjected to a challenge test by i.n. (intranasal) application of OVA (positive control or test groups) or PBS (negative control). 48 hours after i.n. application of OVA, the animals were anaesthetized and the lungs were rinsed with 1 mL of PBS. The cells were separated on Cytospin 3 cytocentrifuge (Shandon). The cells were stained in Diff-Quick (Dade) and the percentage of eosinophils was determined by differential counting of at least 100 cells.

Beclomethasone (Pliva d.d.) were used as a standard substances, with positive and negative control. The compounds were administered daily i.n. or i.p. in different doses 2 days before the challenge test and up to the completion of the test.

Corticosterone levels were determined in plasma from each animal using a kit for determination of corticosterone (R&D systems). Compound **Ia** (2 mg/kg intranasaly) had no effect on corticosterone levels, while beclomethasone (1 mg/kg intranasaly) used as standard significantly suppressed corticosterone levels.

Compound **Ia** also statistically significantly reduced (t-test, $p<0.05$) the number of eosinophils in the lung rinse with respect to positive control. It is anticipated that similar results will be observed for other compounds of the disclosure.

**Croton oil induced ear edema in male Sprague-Dawley rats**

Test and reference substances, as well as vehicle (acetone), were administered topically to the inner and the outer surface of the right ear of each animal with an automatic pipette, in a volume of 60μL/ear (30μL/surface), thirty minutes before the croton oil challenge. Test substances were administered in the doses of 2 or 5 mg/ear/60μL of acetone.

Dexamethasone was administered in the dose of 1 mg/ear/60μL of acetone. Thirty minutes later, 20% croton oil emulsion in acetone was applied topically to the inner and the outer surface of the right ear of each animal with an automatic pipette, in a volume of 60μL/ear (30μL/surface). Five hours after the challenge, animals were euthanized by asphyxiation in 100% $CO_2$ atmosphere. For assessing the auricular edema, 8mm discs were cut out of left and right auricular pinna and weighed. The degree of edema was calculated by subtracting the weight of 8 mm disc of the untreated ear from that of the treated contralateral ear. The inhibition of edema in the treated animals was presented as a percentage of that in the control rats (0%).

Dexamethasone, a reference substance, applied topically once (1 mg/ear), significantly reduced ear edema by 85.77% ($p<0.05$, Non-parametric ANOVA, $n = 8$). Compound Ia, a test substance, applied topically once as a single dose of 2 mg/ear reduced the ear edema by 51.58% ($p>0.05$ by Non-parametric ANOVA, $p=0.0285$ by Unpaired t-test, $n = 8$) and applied topically once, in a dose of 5mg/ear, significantly reduced ear edema by 85.53% ($p<0.05$, Non-parametric ANOVA, $n = 8$).

In another experiment compound 17 applied topically once in a dose of 5 mg/ear, significantly reduced ear edema by 76.59% (ANOVA with Tukey-Kramer Multiple Comparisons Test, $p<0.01$, $n = 8$).

**Subcutaneous sponge implantation model**

In this model, local inflammation was followed by determination of newly formed granulation tissue together with determination of compound effects on plasma corticosterone concentration and thymus weight in Sprague-Dawley rats. Sprague - Dawley rats were purchased from Iffa Credo, Lyon, France and 10 weeks old male rats were used. PVA sponge material was purchased as 5 mm thick sheets (Oriolik, Croatia). The sponge sheets were cut into 14 mm disks using appropriate cork bore. The disks were pre-washed first with running tap water, followed by demineralized water

and finally soaked in Pursept solution (Merz Hygiene, Germany) for 1 hour. Afterwards, they were rinsed well with demineralized water, dryed and heated at 80°C for 2 hours and stored under sterile conditions. All substances were dissolved in ethanol, applied onto sterile sponges and allowed to dry in the laminar flow cabinet prior to the sponge implantation. Compound Ia was administered in a single dose of 10mg/0.3mL/sponge while beclomethasone dipropionate was applied at 2 or 10 mg/0.3mL/sponge.

On day 0, animals were anaesthetized by inhalation of Isoflurane (Abbott Laboratories Ltd., USA) and 5% oxygen, delivered in an anesthesia induction chamber (Stoelting Co., USA). Gas scavenging was provided using the Fluovac 240V system (International Market Supply, England). Anesthesia was maintained using a gas anesthesia mask (Stoelting Co., USA) and pedal reflex response where checked at intervals. The implantation area was shaved and subsequently disinfected using Pursept solution (MERZ Hygiene, Germany). Using strict aseptic procedure, two 1 cm long skin incisions were made just below the left and right *regio scapularis.* Small subcutaneous pockets were made on the left side of the left wound and the right side of the right wound using blunt forceps. In all animals, sponges with vehicle or dissolved substance applied were inserted on the left side and non-treated ones on the right side. Skin was closed with sutures and disinfected with Pursept solution. On day 7 rats were anaesthetized by intraperitoneal administration of Thiopental (PLIVA; 0,5mL/100g body weight) and exanguinated by puncturing *A. carotis com.*

Blood was collected into Vacutainer tubes (Becton Dickinson, USA) for plasma corticosterone concentration analysis. Corticosterone levels in plasma were measured using R&D Systems kit for quantitative determination of corticosterone. Competitive ELISA was performed with alkaline phosphatase conjugated corticosterone as competitor. Samples were incubated for 2 hours, and after successive washing, substrate was added. After 1h absorbance was measured on 405 nm. OD values are inversely correlated with increasing corticosterone concentrations. Corticosterone concentrations were calculated using calibration curve generated with corticosterone standard concentration dilutions.

For assessing the thymus weight, thymuses were extirpated and weighed using the analytical balance. The reduction of thymus size in treated animals was presented as a percentage of that in control rats (0%).

For assessing the newly formed granulation tissue, sponges were carefully excised and each sponge perforated with sterile surgical sewing suture to keep it on the top of the Falcon tube during centrifugation. Tubes were centrifuged (1000 rpm per 10 minutes). Sponges were put into sterilizer and heated at 60°C to dry. 24 hours later, the sponges were weighed using the analytical balance for measurement of newly formed granulation tissue. The weight of treated sponge was compared to the weight of contra-lateral non-treated sponge of the same animal.

For thymus and sponge weight comparison, one-way Analyses of Variance (ANOVA) with Tukey-Kramer Multiple Comparisons Test was used. For corticosterone concentration comparison non-parametric ANOVA - Kruskal-Wallis Test with Dunn's Multiple Comparisons test was performed. Level of significance was set at $p<0.05$.

Seven days after sponge implantation, compound **Ia**, administered into the sponge at single dose of 10 mg/sponge, significantly reduced granulation tissue formation, in comparison to non-treated and vehicle sponges. In comparison to vehicle beclomethasone significantly decreased thymus weight seven days after sponge implantation, at both doses: 2 and 10 mg/sponge while compound **Ia** administered at dose of 10 mg/sponge had no effect on thymus weight. In comparison to vehicle control beclomethasone at dose of 10 mg/sponge significantly decreased plasma corticosterone concentration while compound **Ia** had no significant effect on plasma corticosterone levels.

### Lung neutrophilia induced by bacterial lipopolysaccharide

Male Balb/cJ mice (Iffa Credo, Lyon, France) weighing 25 g were randomly grouped in three groups: test, positive and negative control (10 animals per each group). Vehicle, beclomethasone dipropionate or test substance were applied intranasally (i.n.) to Balb/cJ mice. Thirty minutes later, 60 μl of bacterial lipopolysaccharide (LPS), dissolved in PBS at the concentration of 167 μg/ml, was given i.n. to all groups except the negative control group, which received the same volume of PBS. Animals were sacrificed after 24 hours and bronchoalveolar lavage fluid (BALF) collected for determination of neutrophils and IL-6 and

TNF-α concentration. Cytokines were measured using sandwich ELISA (R&D Systems). Statistical analysis was performed using GraphPad prism software, one-way ANOVA Turkey Kramer Multiple comparison test. Beclomethasone dipropionate (2 mg/kg) non-significantly decreased all tested parameters. Compared to positive control group, compound **Ia** in the form of phosphate salt (4 mg/kg) statistically significantly decreased neutrophils and concentrations of TNF-α and IL-6 in BALF.

## SYNTHETIC METHODS AND EXAMPLES

PRECURSORS

In the following examples of methods of preparation, which in no way limit the uniqueness of the invention and disclosure, the synthesis of the compound of Formula I from macrolide precursors **M1-M8** and steroid precursors **S1-**

**S24** and nonsteroidal precursors **D10, D11** and **D12** is described.

Macrolide subunits

Macrolide subunits **M1-M8** are compounds represented by the following general structure:

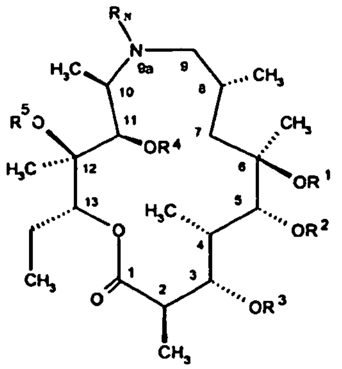

**Table** 1

| | $R_N$ | $R^4$ | $R^5$ | Molecular formula | MH + |
|---|---|---|---|---|---|
| **M1** | H | H | H | $C_{21}H_{41}NO_7$ | 420,2 |
| **M2** | $CH_2CH_2CN$ | H | H | $C_{24}H_{44}N_2O_7$ | 473,3 |
| **M3** | $CH_2-(CH_2)_2-NH_2$ | H | H | $C_{24}H_{48}N_2O_7$ | 477,4 |
| **M4** | $CH_3$ | H | H | $C_{22}H_{43}NO_7$ | 434,7 |
| **M5** | $CH_3$ | >C=O | | $C_{23}H_{41}NO_8$ | 460,4 |
| **M6** | $CH_3$ | $C-(O)-NH-(CH_2)_4-NH_2$ | H | $C_{27}H_{53}N_3O_8$ | 548,4 |
| **M7** | H | >C=O | | $CnH_{39}NO_8$ | 446,5 |
| **M8** | H | $C-(O)-NH-(CH_2)_4-NH_2$ | H | $C_{21}H_{51}N3O_8$ | 534,4 |
| $R^1=R^2=R^3=H$ | | | | | |

Method A

a) Compound **M1** (480 mg; 1.1 mmol) was dissolved in 10 mL of acrylonitrile and the reaction mixture was heated at 95 °C for 24 hours. Subsequently, the solvent was evaporated under reduced pressure. 500 mg of the compound **M2** was obtained, which was used for further synthesis without previous purification.

b) Compound **M2** (500 mg) was dissolved in 20 mL of absolute ethanol and hydrogenated with the catalyst $PtO_2$ (60 mg) for two days at the pressure of 40 atm. The mixture was purified was purified on a silica gel column, eluent $CHCl_3$:MeOH:$NH_4OH$=6:1:0.1.193 mg of compound **M3** was obtained. The properties of compounds **M1, M2** and **M3** are given in Table 1.

Method B

a) Azithromycin (11 g) was dissolved in 40 mL $CHCl_3$ and 80 mL 6M HCl and the reaction mixture was heated at 60 °C for 20 hours. Organic and aqueous layers were separated and then the pH of the aqueous phase was adjusted

to 9.5 and the aqueous layer was then extracted with dichloromethane. The organic layer was dried over anhydrous $Na_2SO_4$ and evaporated. 6 g of compound **M4** was isolated. MS (MH+) = 434.7

b) Compound **M4** (5.95 g, 13.7 mmol), ethylene carbonate (7.3 g, 82.5 mmol) and pottasium carbonate (2,28 g, 16,5 mmol) were mixed in 100 mL ethyl acetate and heated at 75°C under reflux for 72 h. Organic and aqueous layers were separated and the organic layer was dried over anhydrous $Na_2SO_4$ and evaporated. The mixture was purified on a silica gel column in the solvent system $CHCl_3$ : MeOH : $NR_4OH$ = 6:1:0.1.. 3.5 mg of compound **M5** was isolated. MS (*m*/*z*): 460.4 $[MH]^+$.

c) In 1.3 mL (12.85 mmol) of 1,4-diaminobutane, 118 mg (0.26 mmole) of compound **M5** was dissolved. Then, 30 mg (0.26 mmole) of pyridine hydrochloride was added to the solution. The reaction mixture was stirred at room temperature for 20 hours. The product was extracted by dichloromethane and washed with water and the organic layer was subsequently dried over $Na_2SO_4$ and the solvent evaporated under reduced pressure. After purification of the mixture on a silica gel column in the solvent system $CH_2Cl_2$: MeOH: $NH_4OH$= 30: 50: 2, 50 mg of the amine **M6** was obtained. MS ( $MH^+$) = 548.4

d) Compound **M1** (5.05 g, 12.04 mmol) was mixed with ethylencarbonate (6.5 g, 7.38 mmol) and potassium-carbonate (1.7 g, 1.23 mmol). To the reaction mixture, ethyl acetate (90 ml) was added. The solution was heated to 75 °C under stirring for 24 hours. The mixture was washed with water (2x50 ml). Organic layer was then diluted with water (50 ml), adjusted to pH 7 with 2 M HCl and separated. Organic layer was again diluted with water (50 ml), adjusted to pH 7 with 2 M HCl and separated. The organic layers were combined, dried over anhydrous sodium sulphate, filtered and concentrated under vacuum. 1.7 g of the compound **M7** was obtained. MS(ES) m/z: $[MH]^+$ 446.31

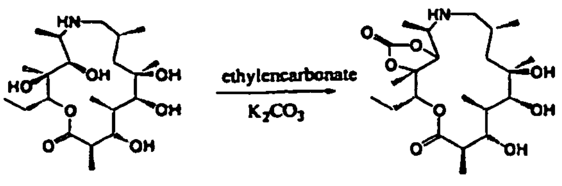


M1 M7

e) In 2,25 ml (22.39 mmol) of 1,4-diaminobutane, 200 mg ( 0.45 mmole) of compound **M7** was dissolved. Then, 52 mg ( 0.45 mmol) of pyridine hydrochloride was added to the solution. The reaction mixture was stirred at room temperature for 3 days. The product was extracted by dichloromethane and washed with water and the organic layer was subsequently dried over $Na_2SO_4$ and the solvent evaporated under reduced pressure. After purification of the mixture on a silica gel column in the solvent system $CH_2Cl_2$: MeOH: $NH_4O$= 30: 50: 2, 60 mg of the amine **M8** was obtained. MS ($MH^+$) = 534.4

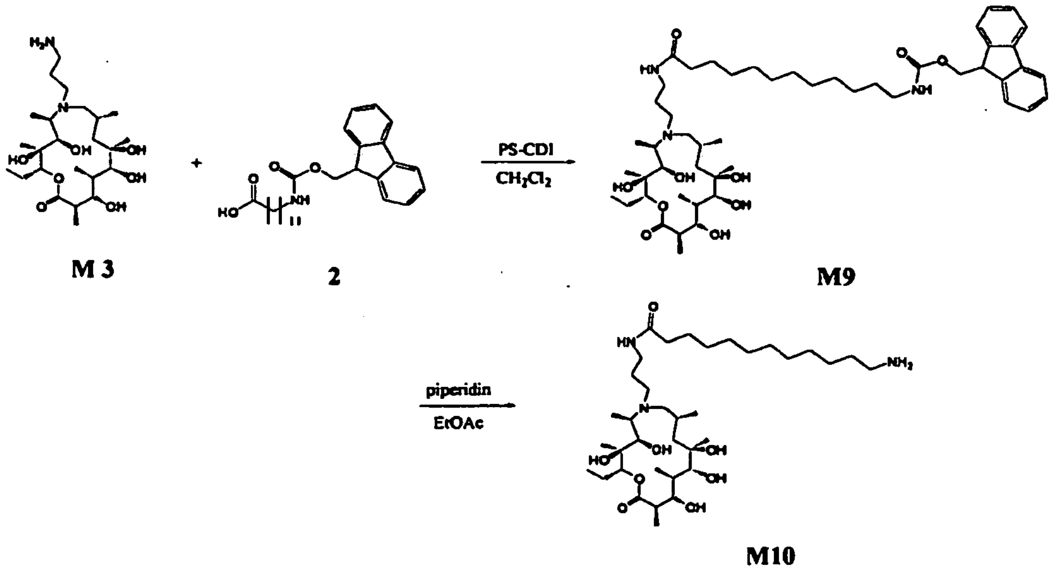


M 3 2 M9

M10

**Compound M9.** PS-Carbodiimide resin (1.15 mg; 1.38 mmol) was added to a dry reaction vessel. 12-(Fmoc-amino)dodecanoic acid (Fluka, Lot 440842/1 50903094) (2) (200 mg,; 0.46 mmol) in $CH_2Cl_2$ (7.5 ml) was added to the dry resin and the mixture stirred at room temperature. After 45 minutes, compound **M3** (109 mg; 0.23 mmol) in $CH_2Cl_2$ (3.5 ml) was added and the reaction stirred at 50°C for 20 hours to afford the amide product. The mixture was filtered and solvent was concentrated under vacuum. Crude product was purified on silica gel column in the solvent system $CH_2Cl_2$:MeOH:$NH_4OH$ 6:1:0.1 the compound **M9** (160 mg) was obtained.
LC/MS (area %): 93.4 %.
HPLC-MS: MS(ES) m/z: $[MH]^+$ 896.66 (calcd. : 896.59)

**Compound M10.** Compound **M9** (160 mg, 0.18 mmol) was dissolved in piperidine (1 ml) and $CH_2Cl_2$ (5 ml). The reaction mixture was stirred at room temperature for 2 hours. The solvents were evaporated and remaining traces of piperidine were removed by addition and removal under vacuum of $CH_2Cl_2$ (several portions). Crude product was purified on silica gel column in the solvent system $CH_2Cl_2$tMeOH:$NH_4OH$ 6:1:0.1 the compound **M10** (132 mg) was obtained.
LC/MS (area %): 95.7 %.
HPLC-MS: MS(ES) m/z: $[MH]^+$ 674.62 (calcd.: 674.52)
IR (KBr) $cm^{-1}$: 3307, 3093, 2927, 2854, 1722, 1715, 1667, 1660, 1651,.1645, 1557, 1539, 1505, 1463, 1456, 1373, 1353, 1265, 1165, 1091, 1053, 958, 811, 736.

Steroid subunits

Steroid subunits **S1-S24** are compounds represented by the following general structure:

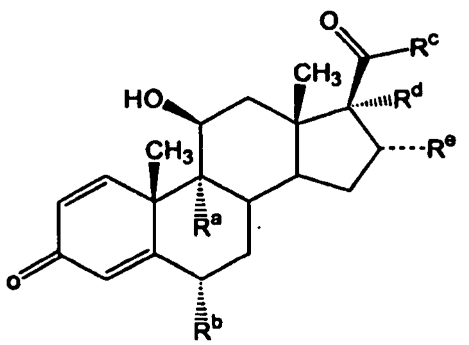

Table 2

| | $R^a$ | $R^b$ | $R^c$ | $R^d$ | $R^c$ | Molecularformula |
|---|---|---|---|---|---|---|
| **S1** | F | H | OH | OH | $CH_3$ | $C_{21}H_{27}FO_5$ |
| **S2** | F | F | OH | OH | $CH_3$ | $C_{21}H_{26}F_2O_5$ |
| **S3** | H | H | $OCH_3$ | OH | H | $C_{21}H_{28}O_5$ |
| **S4** | F | H | OH | OH | H | $C_{20}H_{25}FO_5$ |
| **S5** | H | F | OH | OH | $CH_3$ | $C_{21}H_{27}FO_5$ |
| **S6** | H | $CH_3$ | OH | OH | H | $C_{21}H_{28}O_5$ |
| **S7** | F | H | $NH(CH_2)_2NHBoc$ | H | $CH_3$ | $C_{28}H_{41}FN_2O_5$ |
| **S8** | F | F | OH | DDO | | $C_{23}H_{28}F_2O_6$ |
| **S9** | F | H | OH | OC(O)C=C | $CH_3$ | $C_{24}H_{29}FO_6$ |
| **S10** | F | H | $OCH_3$ | OC(O)C=C | $CH_3$ | $C_{25}H_{31}FO_6$ |
| **S11** | F | F | OH | OC(O)C=C | $CH_3$ | $C_{24}H_{28}F_2O_6$ |
| **S12** | F | F | $OCH_3$ | OC(O)C=C | $CH_3$ | $C_{25}H_{30}F_2O_6$ |
| **S13** | F | H | $NH(CH_2)_2NH_2$ | H | $CH_3$ | $C_{23}H_{33}FN_2O_3$ |
| **S14** | F | H | $OCH_3$ | $O(C)O(CH_2)_2NH(CH_2)_2NHBoc$ | $CH_3$ | $C_{32}H_{47}FN_2O_8$ |
| **S15** | H | F | OH | OC(O)C=C | $CH_3$ | $C_{24}H_{29}FO_6$ |
| **S16** | H | H | OH | OH | H | $C_{21}H2_7FO_5$ |
| **S17** | H | H | OH | OC(O)C=C | H | $C_{24}H_{30}O_6$ |
| **S18** | H | F | $OCH_3$ | OC(O)C=C | $CH_3$ | $C_{25}H_{31}FO_6$ |
| **S19** | H | H | $OCH_3$ | OC(O)C=C | H | $C_{24}H_{30}O_6$ |
| **S20** | F | H | $S(O)N(CH_3)_2$ | OC(O)C=C | H | $C_{27}H_{34}FNO_6S$ |
| **S21** | F | H | $O(O)N(CH_3)_2$ | OC(O)C=C | H | $C_{27}H_{34}FNO_7$ |
| **S22** | F | H | $OCH_3$ | $O(C)O(CH_2)_2NH(CH_2)_2NH_2$ | $CH_3$ | $C_{27}H_{39}FN_2O_6$ |
| **S23** | H | $CH_3$ | OH | OC(O)C=C | H | $C_{24}H_{30}O_6$ |
| **S24** | H | $CH_3$ | $OCH_3$ | OC(O)C=C | H | $C_{25}H_{32}O_6$ |
| DDO = 2,2-dimethyl-1,3-dioxazolone | | | | | | |

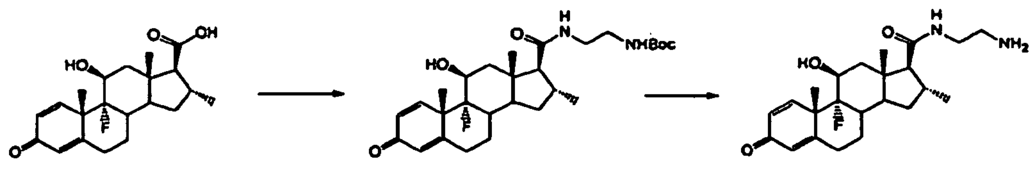

S4 S7 S13

**Scheme 1**

Preparation of S7 (Scheme 1)

To a solution of compound **S4** (500 mg, 1.38 mmol) in dry $CH_2Cl_2$ (10 ml) under argon, were added triethylamine

(1.5 mL, 10.76 mmol), 1-hydroxybenzotriazole (373 mg, 2.76 mmol), $NH_2CH_2CH_2NHBoc$ (218 μl, 1.38 mmol) and 1-(3-dimethylaminopropyl)-3-ethylcarbodiimide hydrochloride (1.06 g, 5.52 mmol). The reaction mixture was stirred for 24 hours at room temperature in a flow of argon and concentrated under reduced pressure. Purification on a silica gel column (eluent: $CH_2Cl_2$:MeOH:$NH_4OH$=6:1:0.1) gave 646 mg of the compound **S7.**
MS(ES) m/z: $[MH]^+$ 505.46

Preparation of **S13** (Scheme 1)

A solution of compound **S7** (630 mg, 1.25 mmol) in TFA (3 ml) and $CH_2Cl_2$ (3 ml) was stirred at room temperature for 1,5 hours. TFA and $CH_2Cl_2$ was removed under vacuum, and remaining traces of TFA were removed by addition and removal under vacuum of $CH_2Cl_2$ (several portions) to give 1.32 g of the compound **S13.**
MS(ES) m/z: $[MH]^+$ 405.30

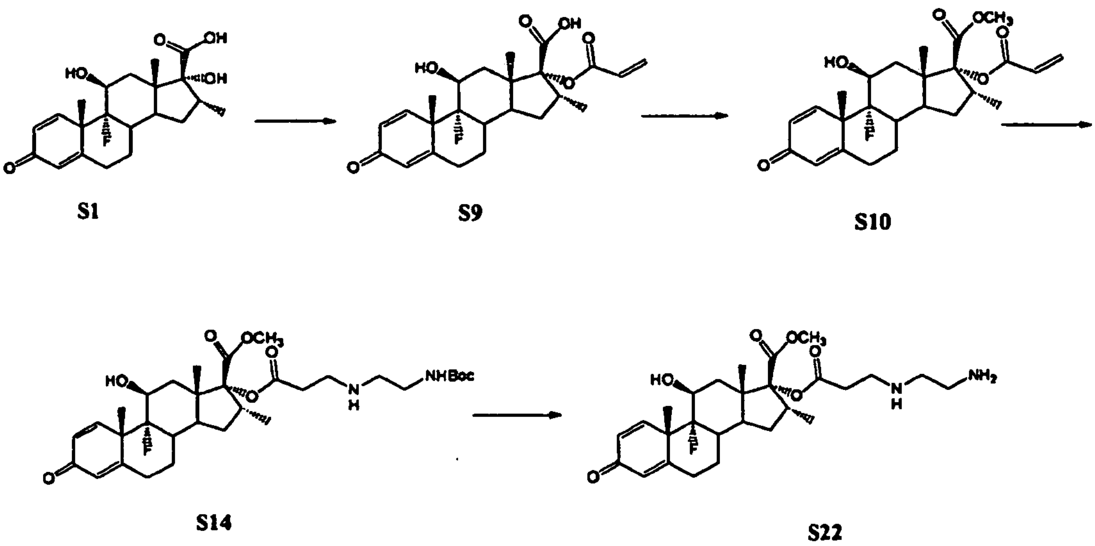

S1 S9 S10

S14 S22

**Scheme 2**

Preparation of **S9** (Scheme 2)

A solution of steroid **S1** (1.5 g, 3.96 mmol) and triethylamine (1.1 ml, 7.93 mmol) in $CH_2Cl_2$ (40 ml) at 0 °C was treated with acryloyl chloride (644 μl, 7.93 mmol). After 30 min the reaction mixture was washed with sat. $NaHCO_3$ and then $H_2O$, dried over anhydrous $Na_2SO_4$ and evaporated under reduced pressure to give the solid intermediate. This was stirred in acetone (30 mL) with diethylamine (2.07 mL, 19.8 mmol) for 2 hours. Solution was concentrated, diluted with water and washed with ethyl acetate. The aqueous phase was acidified to pH 2 with 2 M HCl and filtered to provide a solid. 1.28 g of the compound **S9** was obtained. MS(ES) m/z: $[MH]^+$ 433.28; IR (KBr) $cm^{-1}$ : 3477, 2940, 2879, 2601, 1731, 1655, 1596, 1452, 1401, 1376, 1276, 1401, 1376, 1276, 1221, 1195, 1146, 1118, 1064, 1038, 1011, 975, 951, 931, 904, 874, 832, 812, 767, 703, 657, 635.

Preparation of **S10** (Scheme 2)

To a solution of compound **S9** (1.1 g, 2.54 mmol) in dry THF (8 ml) under argon, was added LiOHx$H_2O$ (106 mg, 2.54 μmol). Reaction mixture was stirred at room temperature for 30 min. $Me_2SO_4$ (235 μl, 2.54 mmol) was then added and the resulting mixture was stirred at 65 °C for 3 hours. The reaction mixture was diluted with ethyl acetate (30 ml) and washed with sat. $NaHCO_3$ and then with water. Organic layer was dried over $Na_2SO_4$ and the solvent evaporated under reduced pressure. 925 mg of the compound S10 was obtained. MS(ES) m/z: $[MH]^+$ 447.32; IR (KBr) $cm^{-1}$ : 3333, 3111, 2944, 2878, 1753, 1728, 1659, 1604, 1450, 1434, 1409, 1285, 1262, 1239, 1192, 1148, 1117, 1101, 1065, 1043, 1013, 977, 928, 893, 879, 809, 707, 686, 662.

Preparation of **S14** (Scheme 2)

In a solution of compound **S10** (800 mg, 1.8 mmol) in MeOH ( 20 ml) and $CH_3CN$ (10 ml) $NH_2CH_2CH_2NHBoc$ (570 μl, 3.6 mmol) was added. Reaction mixture was stirred at 55 °C for 24 hours. After evaporation of the solvent under reduced pressure mixture was purified on a silica gel column in the solvent system $CH_2Cl_2$:MeOH:$NH_4OH$=90:9:1.5. 925 mg of the compound S14 was obtained. MS(ES) m/z: $[MH]^+$ 607.38 ; IR (KBr) $cm^{-1}$ : 3404, 2975, 2941, 2878, 1745, 1665, 1619, 1509, 1459, 1392, 1366, 1268, 1243, 1175, 1116, 1064, 1047, 1032, 1015, 977, 929, 889, 814, 795, 735, 706, 687, 656.

Preparation of **S22** (Scheme 2)

A solution of compound **S14** (697 mg, 1.15 mmol) in TFA (2 ml) and $CH_2Cl_2$ (2 ml) was stirred at room temperature for 2 hours. TFA and $CH_2Cl_2$ was removed under vacuum, and remaining traces of TFA were removed by addition and removal under vacuum of $CH_1Cl_2$ (several portions). Crude product was diluted in $CH_2Cl_2$ (20 ml) and extracted with water.

Aqueous layer was neutralised with NaOH and extracted with ethyl acetate (2x20 ml). The organic layers were combined, dried over *$a_2$SO4, filtered and concentrated under vacuum. 523 mg of the compound **S22** was obtained. MS(ES) m/z: $[MH]^+$ 507.44; IR (KBr) $cm^{-1}$ : 3416, 2940, 2878, 1741, 1664, 1619, 1452, 1392, 1375, 1268, 1240, 1201, 1178, 1117, 1064, 1047, 1032, 1014, 977, 928, 889, 799, 721, 686.

Preparation of **S15**

A solution of steroid **S5** ( 500 mg, 1.32 mmol) and triethylamine (0.37 mL, 2.64 mmol) in $CH_2Cl_2$ ( 30 ml) at 0°C was treated with acryloyl chloride (0.22 mL, 2.64 mmol). After 30 min the reaction mixture was diluted with $CH_2Cl_2$, washed with aqueous $NaHCO_3$ and then $H_2O$, dried and evaporated to give the solid intermediate. This was stirred in acetone ( 25 mL) with diethylamine ( 0.69 mL, 6.61 mmmol) for 2 hours. Solution was concentrated, diluted with water and washed with EtOAc.The aqueous phase was acidified to pH 2 with 2 N HCl and filtered to provide a solid. 259 mg of compound **S15** was obtained. MS (*m*/*z*): 433.36 $[MH]^+$; IR($cm^{-1}$)/KBr: 3438, 3246, 2937, 2878, 2656, 1731, 1717, 1663, 1628, 1609, 1458, 1453, 1409, 1365, 1318, 1300, 1278,1260, 1193, 1180, 1118, 1080, 1061, 1038, 988, 971, 928,900,835,807,780,719,706.

Preparation of **S11**

A solution of steroid **S2** ( 0.5 g, 1.26 mmol) and triethylamine (0.35 mL, 2.52 mmol) in $CH_2Cl_2$ ( 30 ml) at 0°C was treated with acryloyl chloride (0.21 mL, 2.52 mmol). After 30 min the reaction mixture was diluted with $CH_2Cl_2$, washed with aqueous $NaHCO_3$ and then $H_2O$, dried and evaporated to give the solid intermediate. This was stirred in acetone ( 25 ml) with diethylamine ( 0.66 mL, 6.31 mmol) for 2 hours. Solution was concentrated, diluted with water and washed with EtOAc.The aqueous phase was acidified to pH 2 with 2 N HCl and filtered to provide a solid. 212 mg of compound **S11** was obtained. MS (*m*/*z*): 451.00 $[MH]^+$; IR($cm^{-1}$)/KBr: 3423, 2941, 2880, 2624, 1726, 1665, 1619, 1609, 1458, 1407, 1377, 1301, 1261, 1234, 1199, 1150, 1120, 1071, 1041, 1028, 993, 978, 937, 899, 851, 808, 777, 710,659.

Preparation of **S12**

1, 8-Diazabicyclo[5.4.0]undec-7-ene ( DBU) ( 1 equiv, 0.11 mmol) was added to a 10% solution of acid **S11** (0.11 mmol, 50 mg) in dimethylcarbonate and the resulting mixture was heated (100°C) for 10 minutes in microwave reactor. Reaction mixture was cooled to room temperature and diluted with $CH_2Cl_2$ and water. The organic layer was dried over $Na_2SO_4$, evaporated and purified on a silica gel column in the solvent system $CH_2Cl_2$:MeOH = 12:1. 39 mg of the compound S12 was obtained. MS (*m*/*z*): 464.96 $[MH]^+$; IR($cm^{-1}$/KBr: 3339, 2978, 2943, 2881, 1736, 1663, 1619, 1606, 1452, 1452, 1432, 1406, 1375, 1285, 1255, 1243, 1214, 1194, 1116, 1103, 1072, 1042, 1006, 990, 978, 933, 896, 851, 812, 735, 712, 685.

Preparation of **S17**

A solution of steroid **S16** ( 0.5 g, 1.44 mmol) and triethylamine (0,40 mL, 2.89 mmol) in $CH_2Cl_2$ ( 30 ml) at 0°C was treated with acryloyl chloride (0.24 mL, 2.89 mmol). After 30 min the reaction mixture was diluted with $CH_2Cl_2$, washed with aqueous $NaHCO_3$ and then $H_2O$, dried and evaporated to give the solid intermediate. This was stirred in acetone ( 25 mL) with diethylamine ( 0.75 mL, 7.22 mmol) for 2 hours. Solution was concentrated, diluted with water and washed with EtOAc.The aqueous phase was acidified to pH 2 with 2 N HCl and filtered to provide a solid. 260 mg of compound **S17** was obtained. MS (*m*/*z*): 401.25 $[MH]^+$; IR($cm^{-1}$)/KBr: 3567, 3448, 2938, 2914, 2851, 2643, 2602,

1732, 1713, 1653, 1606, 1596, 1452, 1406, 1394, 1346, 1297,1258, 1212, 1184, 1126, 1084, 1040, 988, 972, 941, 930, 908, 888, 828, 812, 769, 719, 656.

Preparation of **S18**

1, 8-Diazabicyclo[5.4.0]undec-7-ene ( DBU) ( 1 equiv, 0.23 mmol) was added to a 10% solution of acid **S15** (0.23 mmol, 100 mg) in dimethylcarbonate and the resulting mixture was heated to reflux ( 90°C). After completion, the reaction mixture was cooled to room temperature and diluted with EtOAc and water. The organic layer was dried over $Na_2SO_4$, evaporated and purified on a silica gel column in the solvent system $CH_2Cl_2$:MeOH:$NH_4OH$ = 90:8:1. 40 mg of the compound **S18** was obtained. MS (*m*/*z*): 447.39 $[MH]^+$; IR($cm^{-1}$)/KBr: 3423, 3368, 2973, 2944, 2875, 1737, 1726, 1657, 1619, 1602, 1459, 1450, 1406, 1389, 1367, 1307, 1284, 1242, 1196, 1116, 1083, 1065, 1040, 987, 976, 941, 928, 895, 828, 812, 712, 671.

Preparation of **S19**

1, 8-Diazabicyclo[5.4.0]undec-7-ene ( DBU) ( 1 equiv, 0.25 mmol) was added to a 10% solution of acid S17 (0.25 mmol, 100 mg) in dimethylcarbonate and the resulting mixture was heated to reflux ( 90°C). After completion, the reaction mixture was cooled to room temperature and diluted with EtOAc and water. The organic layer was dried over $Na_2SO_4$, evaporated and purified on a silica gel column in the solvent system $CH_2Cl_2$:MeOH:$NH_4OH$ = 90:8:1. 42 mg of the compound **S19** was obtained.
MS (*m*/*z*): 415.32 $[MH]^+$

Preparation of **S23**

A solution of steroid S6 ( 700 mg, 1.942 mmol) and triethylamine (0.54 mL, 3.884 mmol) in $CH_2Cl_2$ ( 50 ml) at 0°C was treated with acryloyl chloride (0.315 mL, 3.884 mmol). After 30 min the reaction mixture was diluted with $CH_2Cl_2$, washed with aqueous $NaHCO_3$ and then $H_2O$, dried and evaporated to give the solid intermediate. This was stirred in acetone ( 40 mL) with diethylamine ( 1.015 mL, 9.71 mmmol) for 2 hours. Solution was concentrated, diluted with water and washed with EtOAc.The aqueous phase was acidified to pH 2 with 2 N HCl and filtered to provide a solid. 111 mg of compound **S23** was obtained. MS (*m*/*z*): 415.48 $[MH]^+$

Preparation of **S24**

In a solution of compound **S23** ( 100 mg, 0.24 mmol) in dry THF ( 1.5 mL) 10,1 mg (0.24 mmol) of $LiOHxH_2O$ was added. Reaction mixture was stirred at room temperature for 30 min. In the reaction mixture 22.3 μL of $Me_2SO_4$ (0.24 mmol) was added and the mixture was stirred for 2 hours at 65°C. After completion, the reaction mixture was cooled to room temperature, diluted with 20 mL EtOAc and then treated sequentially with 20 mL of saturated $NaHCO_3$ and 20 mL of water. The organic layer was dried over $Na_2SO_4$, and evaporated. 121 mg of compound **S24** was obtained. MS (*m*/*z*): 429.48 $[MH]^+$

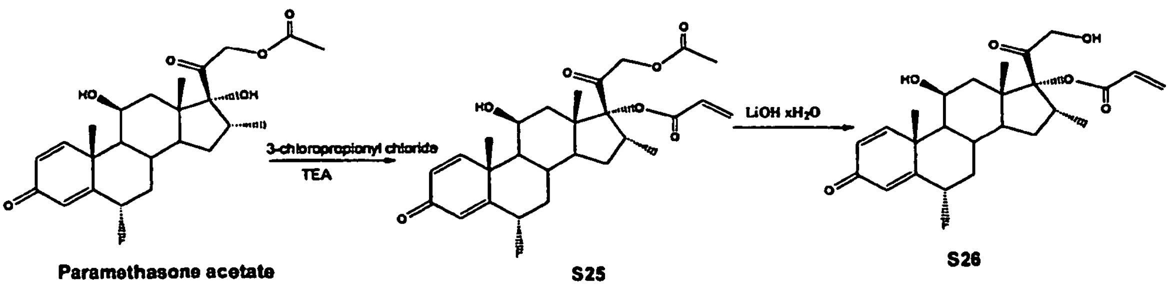


**Paramethasone acetate** **S25** **S26**

**Preparation of compound S25**

To the solution of 1.0 g (2.30 mmol) paramethasone acetate in 20 mL toluene, 960 μL (6.90 mmol, 3eq) TEA was added, followed by 219 μL (2.30 mmol, 1 eq) 3-chloropropionyl chloride. The solution was stirred at room temperature for 24 hours, the evaporated and the crude product purified on the silicagel column, using solvent system ethyl-acetate:

hexane 5:3. 417 mg of pure product was isolated.

MS (*m*/*z*):489.38 $[MH]^+$ MS(teor.)=488.55
Purity (HPLC-MS): 96.42 %
IR (KBr): 3386, 3112, 3037, 2960, 2924, 2876, 1751, 1724, 1679, 1619, 1602, 1501, 1452, 1410, 1388, 1373, 1342, 1318, 1267, 1234, 1198, 1178, 1139, 1120, 1092, 1050, 1028, 1011, 986, 916, 894, 871, 845, 816, 789, 742, 721, 695, 657.

**Preparation of compound S26**

To the solution of 100 mg (0.205 mmol) compound **S25** in u 5 mL THF, 17 mg (0.410 mmol) $LiOHxH_2O$ and 5 mL water was added and the reaction mixture stirred at room temperature for 15 min. pH was then adjusted to 8 using 1M NaOH, and the product extracted with DCM. The organic layer was dried over anhydrous $Na_2SO_4$ and evaporated giving 28 mg of pure product.
MS (*m*/*z*):447.10 $[MH]^+$ MS(teor.)=446.51
Purity (HPLC-MS): 98.37 %

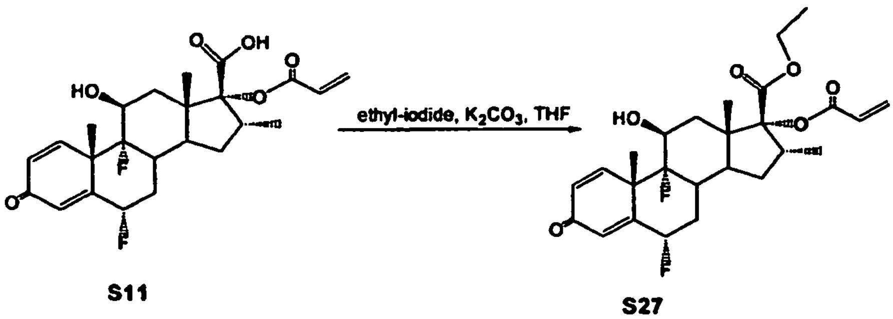


S11 S27

**Compound S27**

A solution of compound **S12** in 10 mL of dry THF was treated with 30.7 mg (1.0 mmol) $K_2CO_3$ and 0,0197mL (1.1 mmol) ethyl-iodide. The reaction mixture was stirred at room temp. for 24 h but no product was obtained. Heating the micture to 55°C resulted with product in 2 hours. The micture was poured into a mixture of 20 mL DCM and 20 mL water and extracted. The organic layer was washed with water, dried over anhydrous $Na_2SO_4$ and evaporated. 45 mg of crude product was isolated.
MS (*m*/*z*):479.09 $[MH]^+$ MS(teor.)=478.53
Purity (HPLC-MS): 76.77 %

**Nonsteroidal subunits**

Precursors for the synthesis are nonsteroidal anti-inflammatory drugs (NSAID), such as aceclofenac, acemetacin, acetaminophen, acetaminosalol, acetyl-salicylic acid, acetyl-salicylic-2-amino-4-picoline-acid, 5-aminoacetylsalicylic acid, alclofenac, amino-profen, amfenac, anileridine, bendazac, benoxaprofen, bermoprofen, α-bisabolol, bromfenac, 5-bromosalicylic , acid acetate, bromosaligenin, bucloxic acid, butibufen, carprofen, cromoglycate, cinmetacin, clidanac, clopirac, sodium diclofenac, diflunisal, ditazole, enfenamic acid, etodolac, etofenamate, felbinac, fenbufen, fenclozic acid, fendosal, fenoprofen, fentiazac, fepradinol, flufenamic acid, flunixin, flunoxaprofen, flurbiprofen, glucametacin, glycol salicylate, ibufenac, ibuprofen, ibuproxam, indomethacin, indoprofen, isofezolac, isoxepac, isoxicam, ketoprofen, ketorolac, lornoxicam, loxoprofen, meclofenamic acid, mefenamic acid, meloxicam, mesalamine, metiazinic acid, mofezolac, montelukast, naproxen, niflumic acid, olsalazine, oxaceprol, oxaprozin, oxyphenbutazone, parsalmide, perisoxal, phenyl-acetyl-salicylate, phenylbutazone, phenylsalicylate, pirazolac, piroxicam, pirprofen, pranoprofen, protizinic acid, salacetamide, salicylamide-O-acetic acid, salicylsulphuric acid, salicin, salicylamide, salsalate, sulindac, suprofen, suxibuzone, tenoxicam, tiaprofenic acid, tiaramide, tinoridine, tolfenamic acid, tolmetin, tropesin, xenbucin, ximoprofen, zaltoprofen, zomepirac, tomoxiprole, zafirlukast, and some example of precursors are flunixin (**D10**), flufenamic acid (**D11**) and celecoxib (**D12**) :

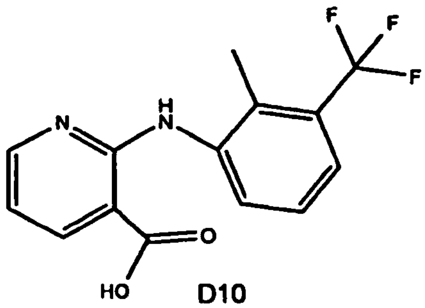

D10

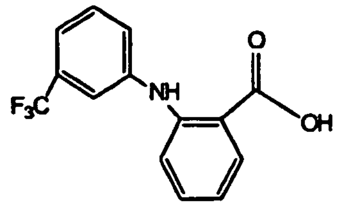

D11

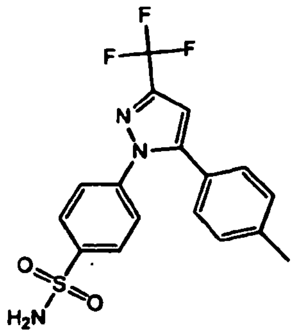

D12

Preparation **of D13** (Scheme 3)

A mixture of celecoxib (**D12**) (4 g, 10.5 mmol), DMAP (640 mg, 5.24 mmol), di-t-butyl dicarbonate (7.52 mL, 31.5 mmol) and triethylamine (1.75 mL, 12.57 mmol) in anhydrous THF (20 mL) was stirred at room temperature for 1 hour. Methyl bromoacetate (2.63 mL, 26.24 mmol) and $K_2CO_3$ (2.9 g, 21 mmol) was then added and the resulting mixture was stirred at room temperature for 22 hours. The reaction mixture was poured into sat. $NaHCO_3$ and extracted with ethyl acetate (2x50 mL). The organic layers were combined, washed with sat. NaCl (50 mL), dried over $MgSO_4$, filtered and concentrated under vacuum. The resulting glass was purified by chromatography (silica gel, 90:9:1.5 $CH_2Cl_2$:MeOH: $NH_4OH$) to afford 4.53 g of the product **D13** as a white powder. MS(ES) m/z: $[MH]^+$ 554.33 ; IR (KBr) $cm^{-1}$ : 3449, 3136, 3108, 2983, 1919, 1759, 1738, 1618, 1598, 1501, 1473, 1450, 1411, 1372, 1314, 1273, 1239, 1165, 1146, 1095, 1016, 995, 976, 939, 846, 808, 763, 744, 718, 653.

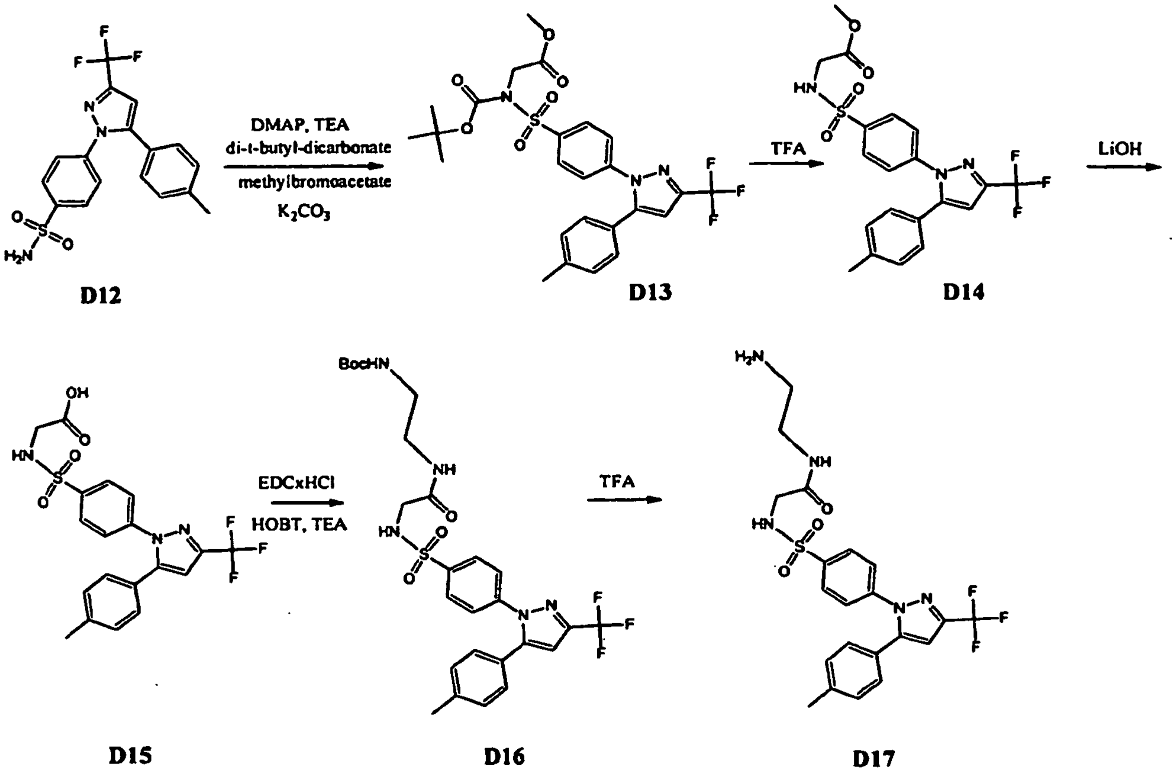


D12 D13 D14

D15 D16 D17

**Scheme 3**

Preparation of **D14** (Scheme 3)

A solution of compound **D13** (4.53 g, 8.18 mmol) in TFA (5 mL) and $CH_2Cl_2$ (5 mL) was stirred at room temperature for 2 hours. TFA and $CH_2Cl_2$ was removed under vacuum, and remaining traces of TFA were removed by addition and removal under vacuum of $CH_2Cl_2$ (several portions) to give 4.43 g oil product **D14.** MS(ES) m/z: $[MH]^+$ 454.27; IR (KBr) $cm^{-1}$: 3281, 2954, 2925, 1747, 1595, 1555, 1499, 1476, 1438, 1411, 1376, 1354, 1324, 1279, 1238, 1216, 1162, 1133, 1100, 1016, 978, 949, 874, 849, 803, 762, 744, 722, 700, 633.

Preparation of **D15** (Scheme 3)

A solution of compound **D14** (4.43 g, 9.77 mmol) in THF (15 mL) was treated with a solution of LiOH (820 mg, 19.54 mmol) in water (15 mL) and stirred for 30 min. THF was removed under vacuum and resulting mixture was adjusted to pH 2 with 0.1 M HCl. Resulting solid was isolated by filtration to give 3.84 g of the compound **D15.** MS(ES) m/z: $[MH]^+$ 440.25; IR (KBr) $cm^{-1}$: 3396, 1606, 1574, 1501, 1472, 1415, 1373, 1326, 1274, 1238, 1169, 1156, 1129, 1098, 1022, 974, 930, 847, 813, 758, 628.

Preparation of **D16** (Scheme 3)

To a solution of compound **D15** (500 mg, 1.14 mmol) in dry $CH_2Cl_2$ (10 mL) under argon, were added triethylamine (1.4 mL, 10 mmol), 1-hydroxybenzotriazole (308 mg, 2.28 mmol), $NH_2CH_2CH_2NHBoc$ (180 μl, 1.14 mmol) and 1-(3-dimethylaminopropyl)-3-ethylcarbodiimide hydrochloride (874mg, 4.56 mmol). The reaction mixture was stirred for 24 hours at room temperature in a flow of argon and concentrated under reduced pressure. Purification on a silica gel column (eluent: $CH_2Cl_2$:MeOH:$NH_4OH$=6:1:0.1) gave 390 mg of the compound **D16.** MS(ES) m/z: $[MH]^+$ 582.33; IR (KBr) $cm^{-1}$: 3378, 2979, 2933, 2878, 1686, 1598, 1528, 1499, 1473, 1450, 1409, 1369, 1341, 1273, 1238, 1163, 1135, 1097, 1006, 976, 843, 827, 808, 761, 744, 719, 694, 627.

Preparation of **D17** (Scheme 3)

A solution of compound **D16** (300 mg, 0.52 mmol) in TFA (3 mL) and $CH_2Cl_2$ (5 mL) was stirred at room temperature for 2 hours. TFA and $CH_2Cl_2$ was removed under vacuum, and remaining traces of TFA were removed by addition and removal under vacuum of $CH_2Cl_2$ (several portions) to give 250 mg of the product **D17.** MS(ES) m/z: $[MH]^+$ 482.19.

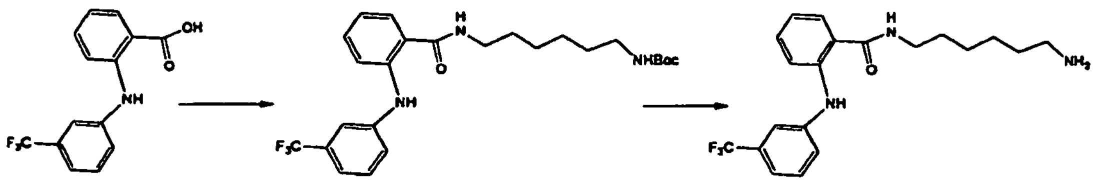

D11 D18 D19

Scheme 4

Preparation of **D18** (Scheme 4)

To a solution of flufenamic acid **D11** (245 mg, 0.87 mmol) in dry $CH_2Cl_2$ (20 ml) under argon, were added triethylamine (1.2 ml, 8.73 mmol), 1-hydroxybenzotriazole (240 mg, 1.78 mmol), $NH_2(CH_2)_6NHFmoc$ (300 mg, 0.9 mmol) and 1-(3-dimethylaminopropyl)-3-ethylcarbodiimide hydrochloride (700 mg, 3.65 mmol). The reaction mixture was stirred for 24 hours at room temperature in a flow of argon and concentrated under reduced pressure. Purification on a silica gel column (eluent: $CH_2Cl_2$:MeOH:$NH_4OH$=6:1:0.1) gave 430 mg of the compound **D18**.

Preparation of **D19** (Scheme 4)

A solution of compound **D18** (400 mg, 0.66 mmol) in ethyl acetate (5 mL) and piperidine (2 mL) was stirred at room temperature for 1 hours. Ethyl acetate and piperidine was removed under vacuum. 370 mg of the compound **D19** was obtained.
MS(ES) m/z: $[MH]^+$ 380.22

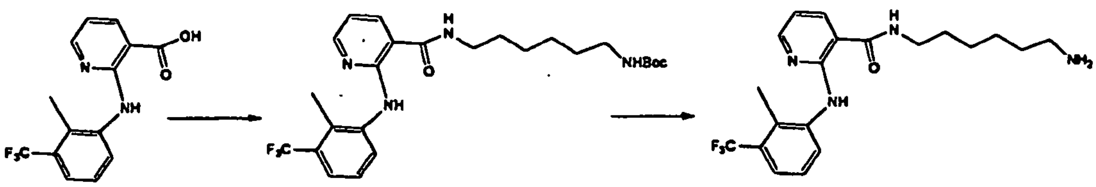

D10 D20 D21

Scheme 5

Preparation of **D20** (Scheme 5)

To a solution of flunixin **D10** (340 mg, 1.15 mmol) in dry $CH_2Cl_2$ (10 mL) under argon, were added triethylamine (1.6 mL, 11.5 mmol), 1-hydroxybenzotriazole (312 mg, 2.3 mmol), $NH_2(CH_2)_6NHFmoc$ (390 mg, 1.15 mmol) and 1-(3-dimethylaminopropyl)-3-ethylcarbodiimide hydrochloride (880 mg, 4.6 mmol)). The reaction mixture was stirred for 24 hours at room temperature in a flow of argon and concentrated under reduced pressure. Purification on a silica gel column (eluent: $CH_2Cl_2$:MeOH:$NH_4OH$=6:1:0.1) gave 548 mg of the compound **D20.**
MS(ES) m/z: $[MH]^+$ 617.66

Preparation **of D21** (Scheme 5)

A solution of compound **D20** (548 mg, 0.89 mmol) in ethyl acetate (5 mL) and piperidine (2 mL) was stirred at room temperature for 1 hours. Ethyl acetate and piperidine was removed under vacuum. 732 mg of the product **D21** was obtained.
MS(ES) m/z: $[MH]^+$ 395.45

**Examples**

**Reference compound 1**

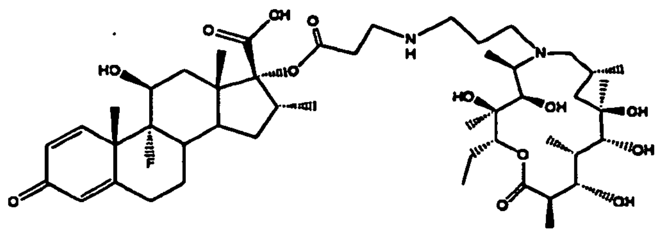

In a solution of compound **S9** ( 250 mg, 0.58 mmol) in methanol (20 mL) 915 mg (1.16 mmol) of the macrolide **M3** was added. Reaction mixture was stirred at 55°C for 24 hours. After evaporation of the solvent, mixture was purified on a silica gel column in the solvent system $CH_2Cl_2$:MeOH:$NH_4OH$ = 30:50:2. 540 mg of the reference compound 1 was obtained. MS (*m*/*z*): 909,42 $[MH]^+$.

**Reference compound 2**

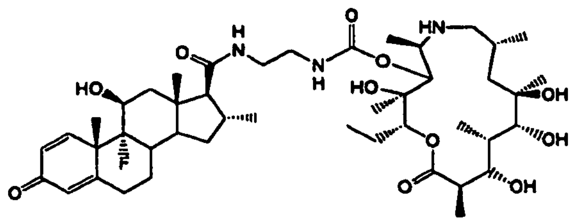

Compound S13 (1.3 g, 3.2 mmol) and compound **M7** (130 mg, 0.3 mmol) was dissolved in pyridine (5 mL). Then, pyridine hydrochloride (35 mg, 0.3 mmol) and 1,8-diazabicyclo [5.4.0]-undec-7-ene (1 ml) were added to the solution. The reaction mixture was stirred at room temperature for 7 days. The product was extracted by $CH_2Cl_2$ and washed with water and the organic layer was subsequently dried over $Na_2SO_4$ and the solvent evaporated under reduced pressure. After purification of the mixture on a silica gel column in the solvent system $CH_2Cl_{21}$MeOH:$NH_4$OH=6: 1 :0.1 80 mg of the reference compound 2 was obtained. MS(ES) m/z: $[MH]^+$ 850.70; IR (KBr) $cm^{-1}$: 3409, 2939, 2877, 1664, 1535, 1495, 1381, 1296, 1248, 1163, 1091, 1070, 975, 928, 889, 829, 757, 702.

**Reference compound 3**

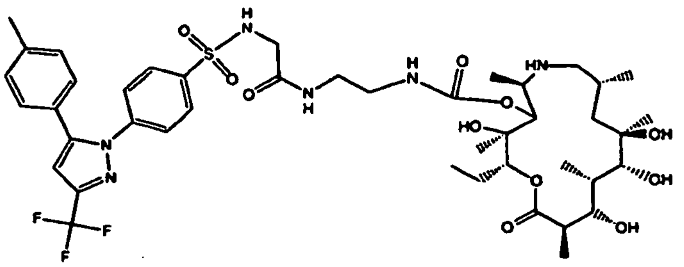

Compound **D17** (220 mg, 0.5 mmol) and compound **M7** (222 mg, 0.5 mmol) was dissolved in pyridine (5 mL). Then, pyridine hydrochloride (58 mg, 0.5 mmol) and 1,8-diazabycyclo [5.4.0]-undec-7-ene (1 mL) were added to the solution. The reaction mixture was stirred at room temperature for 6 days. The product was extracted by $CH_2Cl_2$ and washed with water and the organic layer was subsequently dried over $Na_2SO_4$ and the solvent evaporated under reduced pressure. After purification of the mixture on a silica gel column in the solvent system $CH_2Cl_2$:MeOH:$NH_4OH$+90:9: 1.5 24 mg of the reference compound 3 was obtained. MS(ES) m/z: $[MH]^+$ 927.68; IR (KBr) $cm^{-1}$ : 3416, 2973, 2934, 2880, 1660, 1599, 1546, 1498, 1471, 1376, 1339, 1272, 1238, 1163, 1134, 1097, 974, 843, 808, 761, 740, 703, 615.

**Reference compound 4**

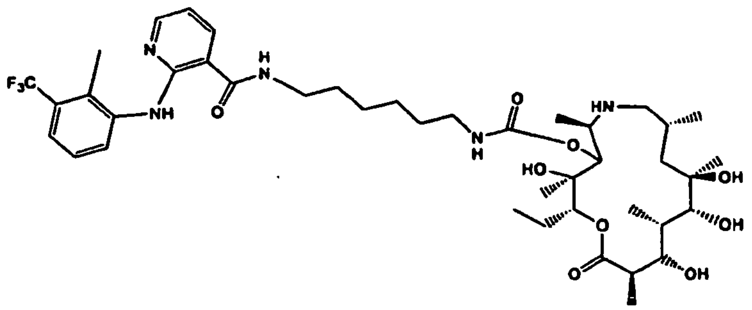

Compound **D21** (732 mg, 1.86 mmol) and compound **M7** (220 mg, 0.5 mmol) was dissolved in pyridine (7 mL). Then, pyridine hydrochloride (60 mg, 0.5 mmol) and 1,8-diazabycyclo [5.4.0]-undec-7-ene (1 mL) were added to the solution. The reaction mixture was stirred at room temperature for 6 days. The product was extracted by $CH_2Cl_2$ and washed with water and the organic layer was subsequently dried over $Na_2SO_4$ and the solvent evaporated under reduced pressure. After purification of the mixture on a silica gel column in the solvent system $CH_2Cl_2$:MeOH:$NH_4OH$=90:9:1.5 70 mg of the reference compound **4** was obtained. MS(ES) m/z: $[MH]^+$ 840.43; IR (KBr) $cm^{-1}$: 3339, 2971, 2935, 2878, 1645, 1593, 1524, 1462, 1380, 1320, 1254, 1186, 1168, 1122, 1088, 1023, 975, 927, 795, 772, 720, 665, 614.

**Reference compound 5**

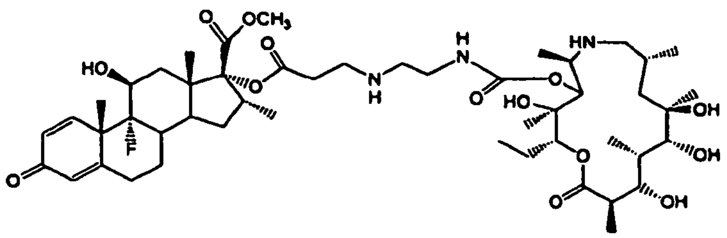

Compound **S22** (220 mg, 0.5 mmol) and compound **M7** (223 mg, 0.5 mmol) was dissolved in pyridine (5 mL).

Then, pyridine hydrochloride (58 mg, 0.5 mmol) and 1,8-diazabycyclo [5.4.0]-undec-7-ene (1 mL) was added to the solution. The reaction mixture was stirred at room temperature for 6 days. The product was extracted by $CH_2Cl_2$ and washed with water and the organic layer was subsequently dried over $Na_2SO_4$ and the solvent evaporated under reduced pressure. After purification of the mixture on a silica gel column in the solvent system $CH_2Cl_2$:MeOH:$NH_4OH$=90:9:1.5 80 mg of the reference compound 5 was obtained. MS(ES) m/z: $[MH]^+$ 953.80.

**Reference compound 6**

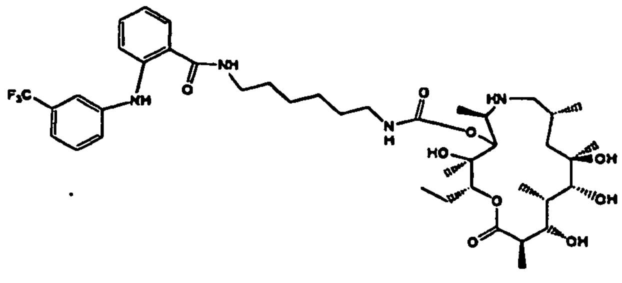

Compound **D19** (380 mg, 0.5 mmol) and compound **M7** (222 mg, 0.5 mmol) was dissolved in pyridine (5 mL). Then, pyridine hydrochloride (58 mg, 0.5 mmol) and 1,8-diazabycyclo [5.4.0]-undec-7-ene (1 mL) were added to the solution. The reaction mixture was stirred at room temperature for 6 days. The product was extracted by $CH_2Cl_2$ and washed with water and the organic layer was subsequently dried over $Na_2SO_4$ and the solvent evaporated under reduced pressure. After purification of the mixture on a silica gel column in the solvent system $CH_2Cl_2$:MeOH:$NH_4OH$=90:9:1.5 18 mg of the reference compound 6 was obtained. MS(ES) m/z: $[MH]^+$ 825.47; IR (KBr) $cm^{-1}$: 3369, 2970, 2935, 2878, 1632, 1595, 1524, 1452, 1426, 1377, 1336, 1283, 1248, 1165, 1124, 1097, 1070, 975, 928, 793, 750, 699, 663.

**Reference compound 7**

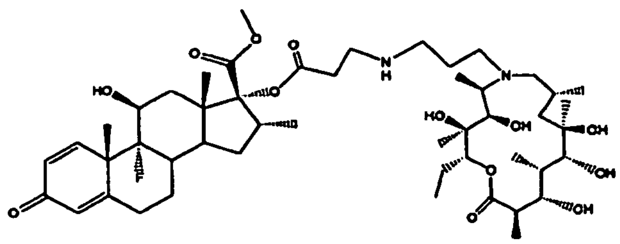

In a solution of compound **S10** ( 50 mg, 0.11 mmol) in methanol ( 10 mL) 107 mg ( 0.22 mmol) of the macrolide **M3** was added. Reaction mixture was stirred at 55°C for 24 hours. After evaporation of the solvent, mixture was purified on a silica gel column in the solvent system $CH_2Cl_2$:MeOH:$MH_4OH$ = 6:1:0.1. 23 mg of the reference compound 7 was obtained. MS (*m*/*z*): 923.47 $[MH]^+$ ; IR($cm^{-1}$)/KBr: 3449, 2938, 2878, 1738, 1665, 1621, 1562, 1544, 1525, 1521, 1460, 1377, 1353, 1266, 1242, 1178, 1099, 1050, 1015, 977, 957, 891, 810, 705, 673.

**Reference compound 8**

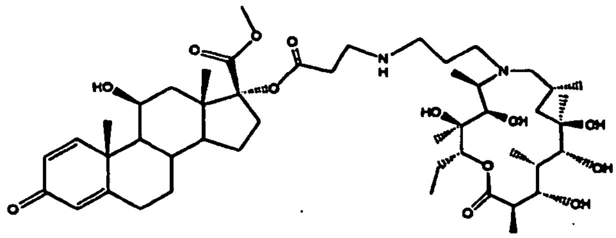

In a solution of compound **S19** ( 41 mg, 0.099 mmol) in methanol ( 6 mL) 47 mg ( 0.009 mmol) of the macrolide **M3** was added. Reaction mixture was stirred at 55°C for 24 hours. After evaporation of the solvent, mixture was purified on a silica gel column in the solvent system $CH_2Cl_2$:MeOH:$NH_4OH$ = 90:8:1. 19 mg of the reference compound 8 was obtained.. MS (*m*/*z*): 891.58 $[MH]^+$; IR($cm^{-1}$)/KBr: 3449, 2974, 2935, 2876, 1871,1846, 1735, 1658, 1618, 1545, 1509, 1459, 1375, 1352, 1177, 1127, 1087, 1036, 995, 958, 939, 888, 819, 708.

**Reference compound 9**

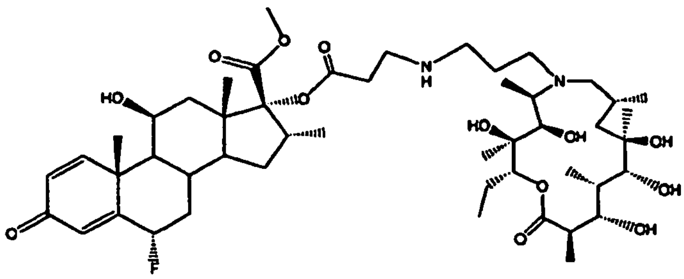

Reaction mixture of compound **S18** ( 77 mg, 0.17 mmol) in methanol ( 8 mL) and 165 mg ( 0.35 mmol) of the macrolide **M3** was stirred at 55°C for 24 hours. After evaporation of the solvent, mixture was purified on a silica gel column in the solvent system $CH_2Cl_2$:MeOH:$NH_4OH$ = 90:8:1 and 36 mg of the reference compound 9 was obtained. MS (*m*/*z*): 923.61 $[MH]^+$; IR($cm^{-1}$)/KBr: 3449, 2974, 2951, 2935, 2878, 1736, 1665, 1626, 1605, 1561, 1509, 1459, 1375, 1319, 1289, 1254, 1175, 1080, 1038, 958, 928, 900, 822, 757, 719, 666.

**Compound Ia**

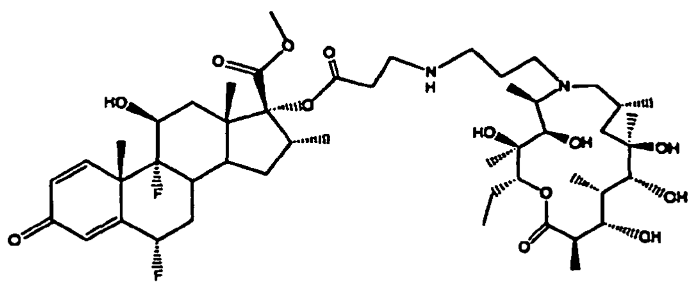

Reaction mixture of compound **S12** ( 37 mg, 0.08 mmol) in methanol:acetonitrile ( 2:5) and 76 mg (0.16 mmol) of the macrolide **M3** was stirred at 55°C for 24 hours. After evaporation of the solvent, mixture was purified on a silica gel column in the solvent system **$CH_2Cl_2$:MeOH:$NH_4OH$** = 90:8:1 and 55 mg of the compound **Ia** was obtained. MS *(m/ z)***:** 941.97 $[MH]^+$; IR($cm^{-1}$)/KBr: 3449, 2964, 2936, 2878, 1736, 1670, 1630, 1561, 1509, 1459, 1376, 1288, 1260, 1236, 1178, 1106, 1074, 1035, 994, 956, 940, 899, 849, 817, 755, 709, 669.

**Reference compound 10**

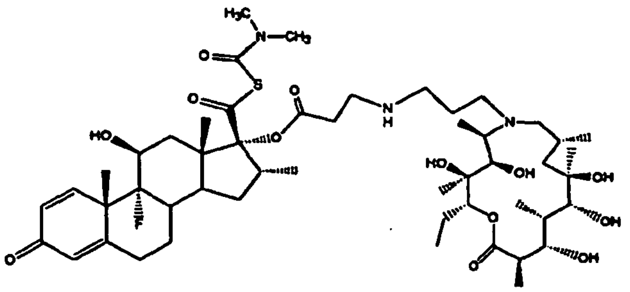

A solution of reference compound **1** (130 mg, 0.14 mmol) and dimethyltiocarbamoylchloride (35.32 mg, 0.286 mmol) in 2-butanone (10 mL) at room temperature was treated sequentially with triethylamine (0.044 ml, 0,31 mmol), sodium iodide (21 mg, 0,143 mmol), and water ( 0.013 mL) and stirred for 3 days. Reaction mixture was then treated sequentially with dimethyacetamide (0.52 mL) and water (3.23 mL); cooled to 0°C, stirred for 2 hours and extracted with EtOAc. Organic layer was dried over $Na_2SO_4$ and evaporated under reduced pressure. Mixture was purified on a silica gel column in the solvent system $CH_2Cl_2$:MeOH:$NH_4OH$ = 90:9:0.5. 32 mg of the reference compound **10** was obtained. MS (*m*/*z*): 996.48[MH]$^+$; IR(cm$^{-1}$)KBr: 3449, 2938, 2878, 1735, 1665, 1624, 1458, 1375, 1250, 1174, 1103, 1056, 1034, 1013, 981, 956, 929, 894, 781, 703, 672.

**Reference compound 11**

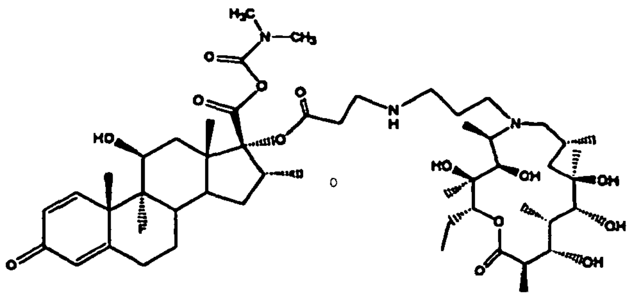

A solution of reference compound **1** (130 mg, 0.14 mmol) and dimethylcarbamoylchloride (35.32 mg, 0.286 mmol) in 2-butanone (10 mL) at room temperature was treated sequentially with triethylamine (0.044 ml, 0.31 mmol), sodium iodide (21 mg, 0.143 mmol), and water (0.013 mL) and stirred for 3 days. Reaction mixture was then treated sequentially with dimethyacetamide (0.52 mL) and water (3.23 mL); cooled to 0°C, stirred for 2 hours and extracted with EtOAc. Organic layer was dried over $Na_2SO_4$ and evaporated under reduced pressure. Mixture was purified on a silica gel column in the solvent system $CH_2Cl_2$:MeOH:$NH_4OH$ = 90:9:0.5. 30 mg of the reference compound 11 was obtained. MS (*m*/*z*): 980.5 [MH]$^+$.

**Reference compound 12**

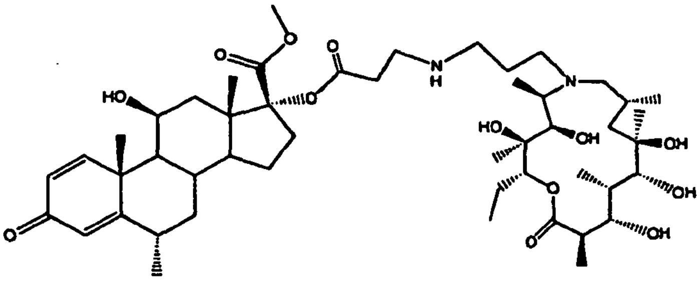

In a solution of compound **S24** ( 70 mg, 0,163 mmol) in 10 mL of methanol and 5 mL of acetonitrile 156 mg ( 0.327 mmol) of the macrolide **M3** was added. Reaction mixture was stirred at 55°C for 24 hours. After evaporation of the solvent, mixture was purified on a silica gel column in the solvent system $CH_2Cl_2$:MeOH:$NH_4OH$ = 90:9:1,5. 50 mg of the reference compound **12** was obtained. MS (*m*/*z*): 906.00 $[MH]^+$.

**Reference compound 13**

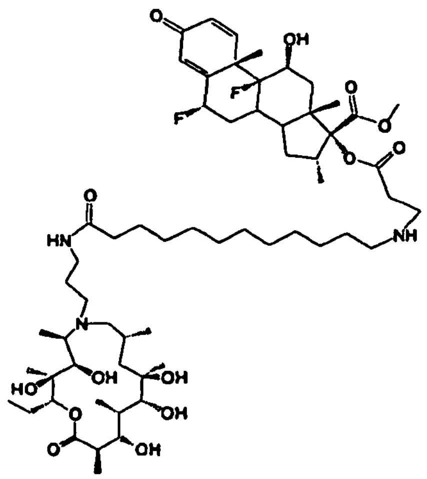

Compound **M10** (60 mg; 0.09 mmol) and compound S12 (42 mg; 0.09 mmol) were dissolved in MeOH (10 ml) and $CH_3CN$ (5 ml) and the resulting reaction mixture was stirred at 50°C over night. After concentrating solvents under vacuum the crude product was purified two times on silica gel column in the solvent system $CH_2Cl_2$:MeOH:$NH_4OH$ 90: 8:1 giving 31 mg of reference compound **13.**
LC/MS (area %): 94 %.
HPLC-MS: MS(ES) m/z: $[MH]^+$ 1138.80 (calcd. : 1138.73)
IR (KBr) $cm^{-1}$: 3417, 2932, 2855, 1742, 1670, 1633, 1547, 1457, 1376, 1288, 1260, 1181, 1091, 1074, 1051, 994, 957, 940, 899, 849, 818, 711.

**Reference compound 14**

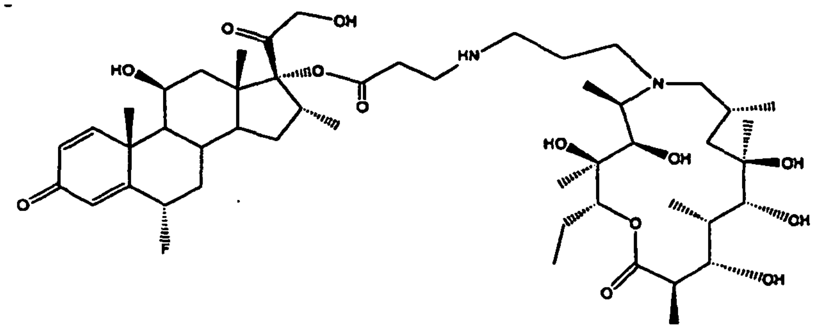

In 10 mL MeOH, 25 mg of compound **S26** was disolved followed by addition 34.68 mg of amine **M3.** The reaction mixture was stirred for 24 h at 55°C. After evaporation of the solvent, product was purified on on a silica gel column using solvent system $CHCl_3$:MeOH:$NH_4OH$ 6:1:0.1 giving 12 mg of reference compound **14.**

MS (*m*/*z*):923.31 $[MH]^+$ MS(teor.)=923.16
Purity (HPLC-MS): 93.50 %

## Reference compound 15

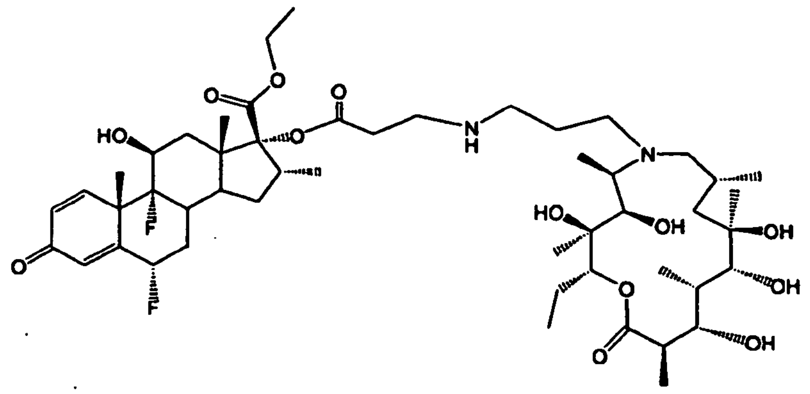

To a solution of 45 mg (0.094 mmol) of compound **S27** in 10 mL MeOH, 89.6 mg (0.188 mmol) of amine **M3** was added. The reaction mixture was stirred for 24 h at 55°C. After evaporation of the solvent, product was purified on on a silica gel column using solvent system $CHCl_3$:MeOH:$NH_4OH$ 6:1:0.1 giving 15 mg of reference compound 15.

MS (*m*/*z*):955.41 $[MH]^+$ MS(teor.)=955.17
Purity (HPLC-MS): 88.26 %

## Reference compound 16

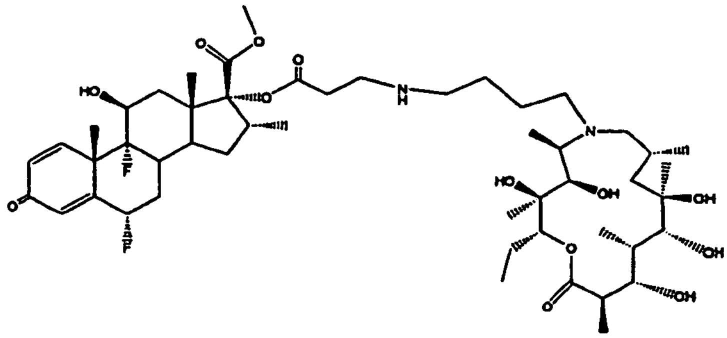

To a solution of 100 mg (0.215 mmol) of compound **S12** in 10 mL MeOH, 137.3 mg (0.280 mmol) amine **M11** (prepared as described in international publication WO2004/094449, example 16) was added. The reaction mixture was stirred for 24 h at 55°C. After evaporation of the solvent, product was purified on a silica gel column using solvent system $CHCl_3$:MeOH:$NH_4OH$ 6:1:0.1 giving 139 mg of reference compound **16.**

MS (*m*/*z*):955.35 $[MH]^+$ MS(teor.)=954.56

Purity (HPLC-MS): 97.65 %

IR (KBr): 3450, 2939, 2879, 1738, 1671, 1628, 1562, 1545, 1525, 1459, 1377, 1288, 1259, 1236, 1179, 1072, 1053, 1036, 994, 957, 941, 900, 850, 817, 756, 709, 667.

**Reference compound 17**

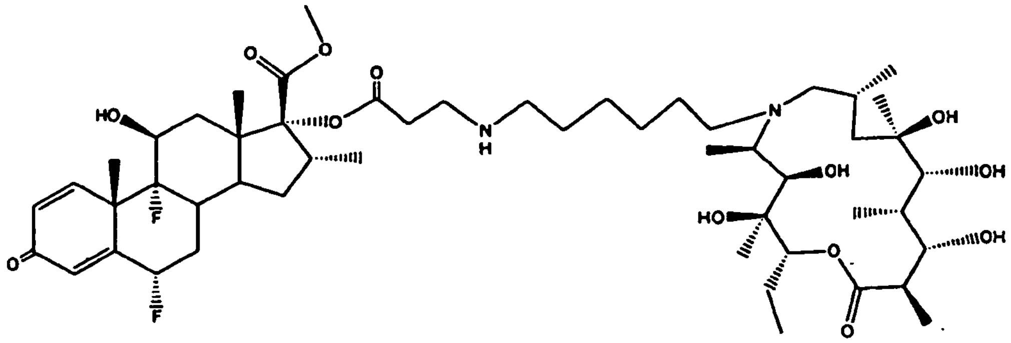

To a solution of 100 mg (0.215 mmol) of compound **S12** in 10 mL MeOH, 137.3 mg (0.280 mmol) of amine **M12** (prepared as described in international publication WO2004/094449, example 17) was added. The reaction mixture was stirred for 24 h at 55°C. After evaporation of the solvent, product was purified on on a silica gel column using solvent system $CHCl_3$:MeOH:$NH_4OH$ 6:1:0.1 giving 162 mg of reference compound **17.**

MS (*m*/*z*):983.37 $[MH]^+$ MS(teor.)=982.59

Purity (HPLC-MS): 98.68 %

IR (KBr): 3448, 2937, 2878, 1736, 1671, 1631, 1458, 1376, 1288, 1259, 1236, 1178, 1105, 1073, 1053, 1036, 994, 975, 957, 941, 899,849, 817, 755, 709, 664.

**Reference compound 18**

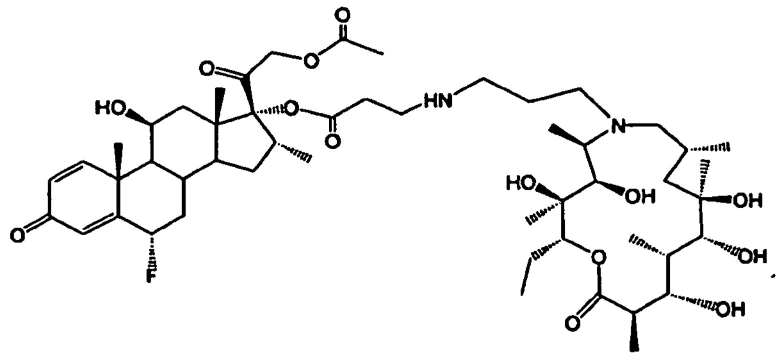

To a solution of 150 mg (0.307 mmol) of compound **S25** in 10 mL MeOH, 292.6 mg (0.615 mmol) of amine **M3** was added. The reaction mixture was stirred for 24 h at 55°C. After evaporation of the solvent, product was purified on on a silica gel column using solvent system $CHCl_3$:MeOH:$NH_4OH$ 6:1:0.1 giving 43 mg of reference compound **18.**
MS (*m/z*):983.37 $[MH]^+$ MS(teor.)=965.19
Purity (HPLC-MS): 98.15 %

**Reference compound 19**

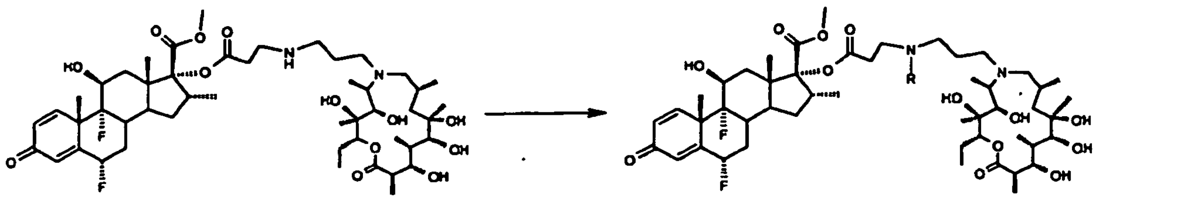

Ia

19 R=$COCH_3$
20 R=$CH_2CH_3$
21 R=$CH(CH_3)_2$
22 R=$CH_2COOCH_3$

Compound **Ia** (97 mg; 0,1 mmol) was dissolved in MeOH (10 ml), and cooled to 2°C. At this temperature, acetanhydride (2 µl; 0,2 mmol) was added drop wise to the reaction mixture. After stirring for three hours at this temperature, the solvent was evaporated and a white, oily product was obtained which was subsequently purified on a silica gel column, eluent $CHCl_3$:MeOH:$NH_4OH$ 90:8:1. 88 mg of the reference compound **19** was obtained.
HPLC-MS: MS(ES) m/z: $[MH]^+$ 983,5
IR (KBr) $cm^{-1}$ : 3444, 2953, 2879, 1744, 1714, 1671, 1633, 1455, 1377, 1289, 1259, 1180, 1074, 1049, 1035, 994, 957, 940, 899, 849, 818, 709.

**Reference compound 20**

Compound **Ia** (100 mg; 0,1 mmol) was dissolved in MeOH (10 ml). In the solution N,N-diisopropylethylamine (177 µl; 1 mmol) and iodoethane (52 µl; 0,65 mmol) were added. The reaction mixture was stirred at 50°C for 24 hours. The solvent was evaporated under vacuum and crude product was purified on silica gel column in the solvent system $CH_2Cl_2$:MeOH:$NH_4OH$ 90:8:1. 22 mg of the reference compound **20** was obtained.
MS(ES) m/z: $[MH]^+$ 969,4.

**Reference compound 21**

Compound Ia (200 mg; 0,2 mmol) was dissolved in $CH_3CN$ (10 ml). In the solution N,N-diisopropylethylamine (442 µl; 2,6 mmol) and 2-iodopropane (520 µl; 5,2 mmol) were added. The reaction mixture was stirred at 50, °C for 24 hours. The solvent was evaporated under vacuum and crude product was purified on silica gel column in the solvent system $CH_2Cl_2$:MeOH:$NH_4OH$ 90:8:1. 70 mg of the reference compound **21** was obtained.
MS(ES) m/z: $[MH]^+$ 983,5.

**Reference compound 22**

Compound **Ia** (200 mg; 0,2 mmol) was dissolved in THF (10 ml). In the solution methylbromoacetate (46 μl; 0,5 mmol) and potassium carbonate (55 mg; 0,4 mmol) were added. The reaction mixture was stirred at room temperature for 20 hours. The solvent was evaporated under vacuum and crude product was purified on silica gel column in the solvent system $CH_2Cl_2$:MeOH:$NH_4OH$ 90:8:1. 148 mg of the reference compound **22** was obtained.
MS(ES) m/z: $[MH]^+$ 1013,5.

## Claims

1. A compound having the structure

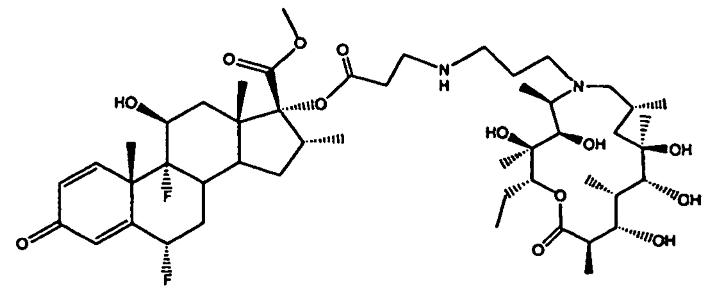
,

or a pharmaceutically acceptable salt or solvate thereof.

2. A compound according to claim 1 having the structure

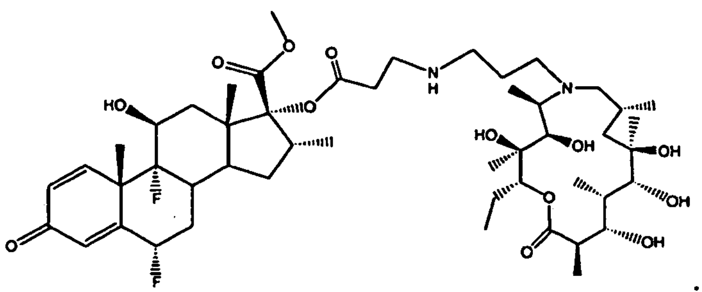
.

3. A pharmaceutical composition comprising a compound according to any one of claims 1 to 2 or a pharmaceutically acceptable salt or solvate thereof as well as pharmaceutically acceptable diluent or carrier.

4. Use of a compound according to any one of claims 1 to 2 or a pharmaceutically acceptable salt or solvate thereof in the manufacture of a medicament for the treatment of inflammatory diseases, disorders and conditions **characterized by** or associated with an undesirable inflammatory immune response, and all diseases and conditions induced by or associated with an excessive secretion of TNF-α and IL-1.

5. Use of a compound according to any one of claims 1 to 2 or a pharmaceutically acceptable salt or solvate thereof in the manufacture of a medicament for the treatment of inflammatory conditions and immune or anaphylactic disorders associated with infiltration of leukocytes into inflamed tissue.

6. Use according to claim 5, wherein inflammatory conditions and immune disorders are selected from the group consisting of asthma, adult respiratory distress syndrome, chronic obstructive pulmonary disease, inflammatory bowel conditions, Crohn's disease, bronchitis and cystic fibrosis.

7. Use according to claim 4, wherein said inflammatory conditions and immune disorders are selected from the group consisting of inflammatory conditions or immune disorders of the lungs, joints, eyes, bowel, skin and heart.

8. Use according to claim 4, wherein said inflammatory conditions and immune disorders are selected from the group consisting of asthma, adult respiratory distress syndrome, bronchitis, cystic fibrosis, rheumatoid arthritis, rheumatoid spondylitis, osteoarthritis, gouty arthritis, uveitis, conjunctivitis, inflammatory bowel conditions, Crohn's disease, ulcerative colitis, distal proctitis, psoriasis, eczema, dermatitis, coronary infarct damage, chronic inflammation, endotoxin shock and smooth muscle proliferation disorders.

9. Use of a compound according to any of claims 1 to 2 or a pharmaceutically acceptable salt or solvate thereof in the manufacture of a medicament for the treatment of inflammatory diseases, disorders and conditions **characterized by** or associated by excessive unregulated production of cytokines or inflammatory mediators.

10. A compound as claimed in any of claims 1 to 2 or a pharmaceutically acceptable salt or solvate thereof for use in therapy.

11. Use of a compound according to any one of claims 1 to 2 or a pharmaceutically acceptable salt or solvate thereof in the manufacture of a medicament for the treatment of asthma.

12. Use of a compound according to any one of claims 1 to 2 or a pharmaceutically acceptable salt or solvate thereof in the manufacture of a medicament for the treatment of chronic obstructive pulmonary disease.

13. Use of a compound according to any one of claims 1 to 2 or a pharmaceutically acceptable salt or solvate thereof in the manufacture of a medicament for the treatment of inflammatory bowel disease.

14. Use of a compound according to any one of claims 1 to 2 or a pharmaceutically acceptable salt or solvate thereof in the manufacture of a medicament for the treatment of psoriasis.

15. A compound according to any one of claims 1 to 2 or a pharmaceutically acceptable salt or solvate thereof for use in the treatment of asthma.

16. A compound according to any one of claims 1 to 2 or a pharmaceutically acceptable salt or solvate thereof for use in the treatment of chronic obstructive pulmonary disease.

17. A compound according to any one of claims 1 to 2 or a pharmaceutically acceptable salt or solvate thereof for use in the treatment of inflammatory bowel disease.

18. A compound according to any one of claims 1 to 2 or a pharmaceutically acceptable salt or solvate thereof for use in the treatment of psoriasis.

**Patentansprüche**

1. Eine Verbindung mit der Struktur

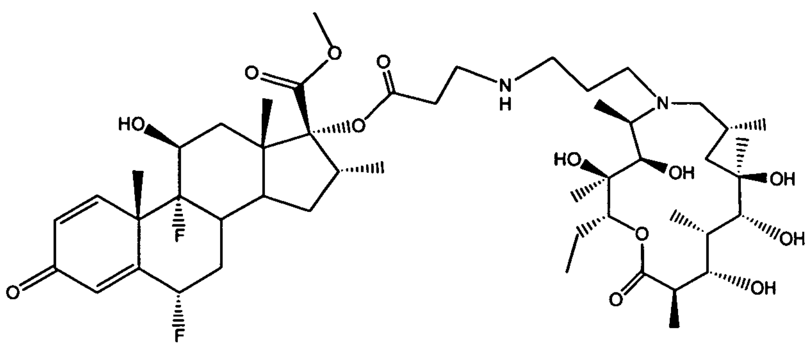

oder ein pharmazeutisch verträgliches Salz oder Solvat davon.

2. Eine Verbindung gemäß Anspruch 1 mit der Struktur

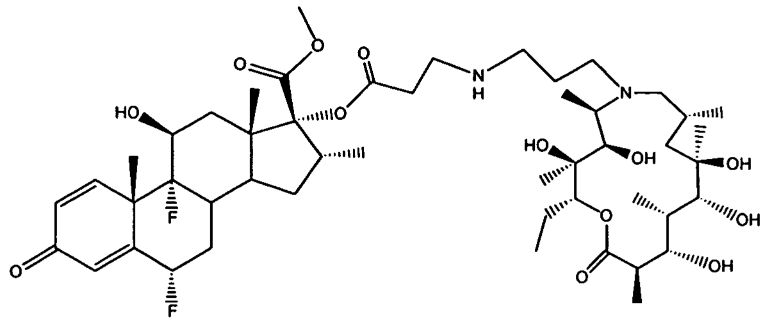

.

3. Ein Arzneimittel, umfassend eine Verbindung gemäß einem der Ansprüche 1 bis 2 oder ein pharmazeutisch verträgliches Salz oder Solvat davon sowie ein pharmazeutisch verträgliches Verdünnungsmittel oder einen pharmazeutisch verträglichen Träger.

4. Verwendung einer Verbindung gemäß einem der Ansprüche 1 bis 2 oder eines pharmazeutisch verträglichen Salzes oder Solvats davon bei der Herstellung eines Medikaments zur Behandlung von Entzündungserkrankungen, -störungen und -zuständen, **gekennzeichnet durch** oder in Verbindung mit einer unerwünschten entzündlichen Immunreaktion, und allen Erkrankungen und Zuständen, die hervorgerufen werden **durch** oder in Verbindung stehen mit einer überhöhten Sekretion von TNF-$\alpha$ und IL-1.

5. Verwendung einer Verbindung gemäß einem der Ansprüche 1 bis 2 oder eines pharmazeutisch verträglichen Salzes oder Solvats davon bei der Herstellung eines Medikaments zur Behandlung von Entzündungszuständen und Immunstörungen oder anaphylaktischen Störungen, welche mit dem Eindringen von Leukozyten in entzündetes Gewebe in Verbindung stehen.

6. Verwendung gemäß Anspruch 5, wobei die Entzündungszustände und die Immunstörungen ausgewählt sind aus der Gruppe bestehend aus Asthma, Atemnotsyndrom bei Erwachsenen, chronisch obstruktiver Lungenerkrankung, entzündlichen Darmerkrankungen, Morbus Crohn, Bronchitis und Mukoviszidose.

7. Verwendung gemäß Anspruch 4, wobei die Entzündungszustände und Immunstörungen ausgewählt sind aus der Gruppe bestehend aus Entzündungszuständen oder Immunstörungen der Lungen, Gelenke, Augen, des Darms, der Haut und des Herzens.

8. Verwendung gemäß Anspruch 4, wobei die Entzündungszustände und Immunstörungen ausgewählt sind aus der Gruppe bestehend aus Asthma, Atemnotsyndrom bei Erwachsenen, Bronchitis, Mukoviszidose, rheumatoider Arthritis, rheumatoider Spondylitis, Osteoarthritis, Gichtarthritis, Uveitis, Bindehautentzündung, entzündlichen Darmerkrankungen, Morbus Crohn, Colitis ulcerosa, distaler Proktitis, Schuppenflechte, Ekzem, Dermatitis, Koronarinfarktschaden, chronischer Entzündung, Endotoxinschock und proliferativen Störungen der glatten Muskulatur.

9. Verwendung einer Verbindung gemäß einem der Ansprüche 1 bis 2 oder eines pharmazeutisch verträglichen Salzes oder Solvats davon bei der Herstellung eines Medikaments zur Behandlung von Entzündungserkrankungen, -störungen und -zuständen, **gekennzeichnet durch** oder in Verbindung mit überhöhter unkontrollierter Produktion von Cytokinen oder Entzündungmediatoren.

10. Eine Verbindung wie in einem der Ansprüche 1 bis 2 beansprucht oder ein pharmazeutisch verträgliches Salz oder Solvat davon zur Verwendung in der Therapie.

11. Verwendung einer Verbindung gemäß einem der Ansprüche 1 bis 2 oder eines pharmazeutisch verträglichen Salzes oder Solvats davon bei der Herstellung eines Medikaments zur Behandlung von Asthma.

12. Verwendung einer Verbindung gemäß einem der Ansprüche 1 bis 2 oder eines pharmazeutisch verträglichen Salzes oder Solvats davon bei der Herstellung eines Medikaments zur Behandlung von chronisch obstruktiver Lungenerkrankung.

13. Verwendung einer Verbindung gemäß einem der Ansprüche 1 bis 2 oder eines pharmazeutisch verträglichen Salzes oder Solvats davon bei der Herstellung eines Medikaments zur Behandlung von entzündlicher Darmerkrankung.

14. Verwendung einer Verbindung gemäß einem der Ansprüche 1 bis 2 oder eines pharmazeutisch verträglichen Salzes oder Solvats davon bei der Herstellung eines Medikaments zur Behandlung von Schuppenflechte.

15. Eine Verbindung gemäß einem der Ansprüche 1 bis 2 oder ein pharmazeutisch verträgliches Salz oder Solvat davon zur Verwendung bei der Behandlung von Asthma.

16. Eine Verbindung gemäß einem der Ansprüche 1 bis 2 oder ein pharmazeutisch verträgliches Salz oder Solvat davon zur Verwendung bei der Behandlung von chronisch obstruktiver Lungenerkrankung.

17. Eine Verbindung gemäß einem der Ansprüche 1 bis 2 oder ein pharmazeutisch verträgliches Salz oder Solvat davon zur Verwendung bei der Behandlung von entzündlicher Darmerkrankung.

18. Eine Verbindung gemäß einem der Ansprüche 1 bis 2 oder ein pharmazeutisch verträgliches Salz oder Solvat davon zur Verwendung bei der Behandlung von Schuppenflechte.

## Revendications

1. Composé ayant la structure

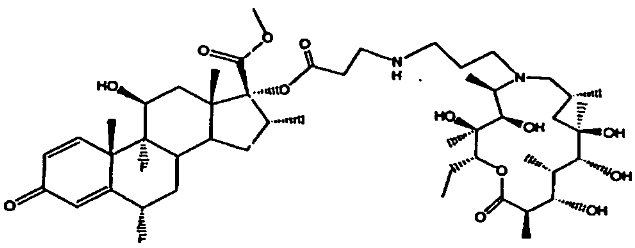

,

ou un de ses sels ou produits de solvatation pharmaceutiquement acceptables.

2. Composé suivant la revendication 1, ayant la structure

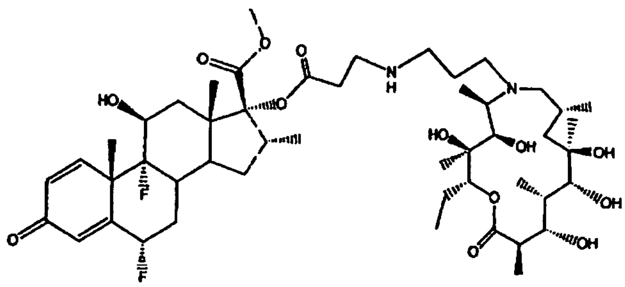

.

3. Composition pharmaceutique comprenant un composé suivant l'une quelconque des revendications 1 et 2 ou un de ses sels ou produits de solvatation pharmaceutiquement acceptables ainsi qu'un diluant ou support pharmaceutiquement acceptable.

4. Utilisation d'un composé suivant l'une quelconque des revendications 1 et 2 ou d'un de ses sels ou produits de

solvatation pharmaceutiquement acceptables dans la production d'un médicament destiné au traitement de maladies, troubles et affections inflammatoires **caractérisés par** ou associés à une réponse immunitaire inflammatoire indésirable, et de toutes les maladies et affections induites par ou associées à une sécrétion excessive de TNF-$\alpha$ et de IL-1.

**5.** Utilisation d'un composé suivant l'une quelconque des revendications 1 et 2 ou d'un de ses sels ou produits de solvatation pharmaceutiquement acceptables dans la production d'un médicament destiné au traitement d'affections inflammatoires et de troubles immunitaires ou anaphylactiques associés à l'infiltration de leucocytes dans un tissu enflammé.

**6.** Utilisation suivant la revendication 5, dans laquelle les affections inflammatoires et troubles immunitaires sont choisis dans le groupe consistant en l'asthme, le syndrome de détresse respiratoire de l'adulte, une maladie pulmonaire obstructive chronique, des affections intestinales inflammatoires, la maladie de Crohn, la bronchite et la fibrose kystiques.

**7.** Utilisation suivant la revendication 4, dans laquelle lesdits troubles immunitaires et affections inflammatoires sont choisis dans le groupe consistant en des affections inflammatoires ou troubles immunitaires des poumons, des articulations, des yeux, de l'intestin, de la peau et du coeur.

**8.** Utilisation suivant la revendication 4, dans laquelle lesdits troubles immunitaires et affections inflammatoires sont choisis dans le groupe consistant en l'asthme, le syndrome de détresse respiratoire de l'adulte, la bronchite, la fibrose kystique, la polyarthrite rhumatoïde, la spondylite rhumatoïde, l'arthrose, l'arthrite goutteuse, l'uvéite, la conjonctivite, des affections intestinales inflammatoires, la maladie de Crohn, la colite ulcérative, la proctite distale, le psoriasis, l'eczéma, la dermatite, une altération par infarctus coronarien, l'inflammation chronique, l'état de choc dû à une endotoxine et des troubles de prolifération du tissu musculaire lisse.

**9.** Utilisation d'un composé suivant l'une quelconque des revendications 1 et 2 ou d'un de ses sels ou produits de solvatation pharmaceutiquement acceptables dans la production d'un médicament destiné au traitement de maladies, troubles et affections inflammatoires **caractérisés par** ou associés à une production excessive non régulée de cytokines ou de médiateurs inflammatoires.

**10.** Composé suivant l'une quelconque des revendications 1 et 2 ou un de ses sels ou produits de solvatation pharmaceutiquement acceptables, destiné à être utilisé en thérapeutique.

**11.** Utilisation d'un composé suivant l'une quelconque des revendications 1 et 2 ou d'un de ses sels ou produits de solvatation pharmaceutiquement acceptables dans la production d'un médicament destiné au traitement de l'asthme.

**12.** Utilisation d'un composé suivant l'une quelconque des revendications 1 et 2 ou d'un de ses sels ou produits de solvatation pharmaceutiquement acceptables dans la production d'un médicament destiné au traitement d'une maladie pulmonaire obstructive chronique.

**13.** Utilisation d'un composé suivant l'une quelconque des revendications 1 et 2 ou d'un de ses sels ou produits de solvatation pharmaceutiquement acceptables dans la production d'un médicament destiné au traitement d'une maladie intestinale inflammatoire.

**14.** Utilisation d'un composé suivant l'une quelconque des revendications 1 et 2 ou d'un de ses sels ou produits de solvatation pharmaceutiquement acceptables dans la production d'un médicament destiné au traitement du psoriasis.

**15.** Composé suivant l'une quelconque des revendications 1 et 2 ou un de ses sels ou produits de solvatation pharmaceutiquement acceptables, destiné à être utilisé dans le traitement de l'asthme.

**16.** Composé suivant l'une quelconque des revendications 1 et 2 ou un de ses sels ou produits de solvatation pharmaceutiquement acceptables, destiné à être utilisé dans le traitement d'une maladie pulmonaire obstructive chronique.

**17.** Composé suivant l'une quelconque des revendications 1 et 2 ou un de ses sels ou produits de solvatation pharmaceutiquement acceptables, destiné à être utilisé dans le traitement d'une maladie intestinale inflammatoire.

**18.** Composé suivant l'une quelconque des revendications 1 et 2 ou un de ses sels ou produits de solvatation pharmaceutiquement acceptables, destiné à être utilisé dans le traitement du psoriasis.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9413690 A
- WO 9414834 A
- WO 9213872 A
- WO 9213873 A
- WO 0042055 A
- EP 0283055 A
- EP 0775489 A
- EP 771564 A
- WO 02055531 A1
- WO 0205531 A
- US 20040014685 A
- WO 04005310 A2
- US 20040077612 A
- US 20040097434 A
- WO 04005309 A
- US 20050080003 A
- US 20040087517 A
- WO 2003070174 A
- US 6297260 B
- WO 0187890 A
- HR 20010018
- WO 02055531 A
- WO 04005310 A
- US 6402733 B
- US 6273086 B
- US 6228346 B
- WO 2004094449 A


### Non-patent literature cited in the description


- **Gladue, R. P. et al.** *Antimicrob. Agents Chemother.,* 1989, vol. 33, 277-282
- **Olsen, K. M. et al.** *Antimicrob. Agents Chemother.,* 1996, vol. 40, 2582-2585
- *J. Antimicrob. Chemother.,* 1998, vol. 41, 37-46
- *J. Antimicrob. Chemother.,* 1992, vol. 30, 339-348
- *J. Immunol.,* 1997, vol. 159, 3395-4005
- *Am. J. Respir. Crit. Care. Med.,* 1997, vol. 156, 266-271
- **Berge S. M. et al.** Pharmaceutical Salts. *J. of Pharma. Sci.,* 1977, vol. 66, 1
- **E.W. Martin.** Remington's Pharmaceutical Sciences
- **Huang C.M. et al.** *Chem.&Bio/.,* 2000, vol. 7, 453-461
- **Mess S. et al.** *Bioorg.&Med Chem.,* 2001, vol. 9, 1279-1291
- **Hess S. et al.** *Bioorg.&Med Chem.,* 2001, vol. 9, 1279-1291
- **Pandori M. W. et al.** *Chem. &Biol.,* 2002, vol. 9, 567-573
- **T.W. Greene ; P.G.M Wuts.** Protective Groups in Organic Synthesis. John Wiley & Son, Inc, 1991
- **P.J. Kocienski.** Protecting Groups. Georg Thieme Verlag, 1994
- **Newman et al.** *Thorax,* 1985, vol. 40, 61-676
- **Berenberg M.** *J. Asthma USA,* 1985, vol. 22, 87-92